# EUROPEAN PATENT APPLICATION

(11) **EP 2 259 059 A1**
(43) Date of publication of application: **08.12.2010**
(21) Application number: 09161526.0
(22) Date of filing: 29.05.2009
(51) Int. Cl.: G01N 33/50

(54) **Means and methods for ovarian cancer prognosis**

(71) Applicant: Atlas Antibodies AB, 106 91 Stockholm (SE)
(72) Inventor: Jirström, Karin, 216 18 Limhamn (SE); Uhlén, Mathias, 182 79 Stocksund (SE); Pontén, Fredrik, 752 37 Uppsala (SE); Brennan, Donal J., 8 Dublin (IE)
(74) Representative: Wirén, Anders

(57) **Abstract**

A method for determining whether a mammalian subject having an ovarian cancer belongs to a first or a second group, wherein the prognosis of subjects of the first group is better than the prognosis of subjects of the second group is provided. The method comprises the steps of: evaluating the amount of HMGCR protein in at least part of a sample earlier obtained from the subject and determining a sample value corresponding to said evaluated amount; comparing said sample value with a predetermined reference value; and if said sample value is higher than said reference value, concluding that the subject belongs to the first group; and if said sample value is lower than or equal to said reference value, concluding that the subject belongs to the second group.

SEQUENCE LISTING

<110>  Atlas Antibodies AB

<120>  MEANS AND METHODS FOR OVARIAN CANCER PROGNOSIS

<130>  21041682

<160>  3

<170>  PatentIn version 3.3

<210>  1
<211>  140
<212>  PRT
<213>  Homo sapiens

<400>  1

Met Ala Gly Ser Ile Gly Gly Tyr Asn Ala His Ala Ala Asn Ile Val
1               5                   10                  15

Thr Ala Ile Tyr Ile Ala Cys Gly Gln Asp Ala Ala Gln Asn Val Gly
            20                  25                  30

Ser Ser Asn Cys Ile Thr Leu Met Glu Ala Ser Gly Pro Thr Asn Glu
        35                  40                  45

Asp Leu Tyr Ile Ser Cys Thr Met Pro Ser Ile Glu Ile Gly Thr Val
    50                  55                  60

Gly Gly Gly Thr Asn Leu Leu Pro Gln Gln Ala Cys Leu Gln Met Leu
65                  70                  75                  80

Gly Val Gln Gly Ala Cys Lys Asp Asn Pro Gly Glu Asn Ala Arg Gln
                85                  90                  95

Leu Ala Arg Ile Val Cys Gly Thr Val Met Ala Gly Glu Leu Ser Leu
            100                 105                 110

Met Ala Ala Leu Ala Ala Gly His Leu Val Lys Ser His Met Ile His
        115                 120                 125

Asn Arg Ser Lys Ile Asn Leu Gln Asp Leu Gln Gly
    130                 135                 140

<210>  2
<211>  888
<212>  PRT
<213>  Homo sapiens

<400>  2

<210>  3
<211>  14
<212>  PRT
<213>  Homo sapiens

<400>  3

Cys Lys Asp Asn Pro Gly Glu Asn Ala Arg Gln Leu Ala Arg
1               5                   10

## Description

### Field of the invention

The present invention relates to the field of ovarian cancer prognostics and ovarian cancer treatment.

### Background of the invention

### Cancer

Cancer is one of the most common causes of disease and death in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelium and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of a tissue section taken from a tumor remains the golden standard for determining a diagnosis of cancer. For example, for microscopic diagnosis, biopsy material from suspected tumors is collected and examined under the microscope. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical (IHC) methods. The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1 , N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, for most tumor types there is also a classification system to grade the level of malignancy. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is crucial for a correct diagnosis and may provide an instrument to predict a prognosis. The diagnostic and prognostic information for a specific tumor subsequently determines an adequate therapeutic strategy for a given cancer patient. A commonly used method, in addition to histochemical staining of tissue sections, to obtain more information regarding a tumor is immunohistochemical staining. IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be involved in supporting the accurate diagnosis, including staging and grading, of a primary tumor as well as in the diagnostics of metastases of unknown origin. The most commonly used antibodies in clinical practice today include antibodies against cell type "specific" proteins, e.g., PSA (prostate), MelanA (melanocytes) and Thyroglobulin (thyroid gland), and antibodies recognizing intermediate filaments (epithelial, mesenchymal, glial), cluster of differentiation (CD) antigens (hematopoetic, sub-classification of lympoid cells) and markers of malignant potential, e.g., Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites all add relevant information. However, most of these analyses still represent basic research and have yet to be evaluated and standardized for the use in clinical medicine.

### Epithelial ovarian cancer (EOC)

Ovarian cancer, including epithelial ovarian cancer (EOC) is one of the most common forms of human cancer worldwide and a leading cause of death from gynecological malignancy in women. Data from the GLOBOCAN 2002 database presented by Parkin *et al* show that around 0.2 million new cases of ovarian cancer are identified yearly, with the highest incidence rates in developed countries (Parkin DM et al (2005) CA Cancer J Clin 55, 74-108). Further, the incidence of ovarian cancer in the world is approximately 4.0 % of all cancers, and ovarian cancer constitutes the seventh most common cause of death from cancer in women.

Today, early detection and surgery with excision of the tumor is normally of critical importance for a successful treatment. Treatment usually involves surgery, chemotherapy and sometimes radiotherapy. Currently standard postoperative chemotherapy is with platinum in combination with paclitaxel. Unfortunately, relapse is common and based on time to relapse patients are divided into two groups: platinum-sensitive and platinum-resistant. If resistance is suspected, then alternative treatment regimens could be considered such as liposomal doxorubicin, topotecan, gemcitabine or docetaxel.

Ovarian cancer tumors are categorized into stage I-IV (1-4) according to the Federation of Gynecology and Obstetrics (FIGO) classification, a system similar to the TNM staging system. At stage I, the tumor is limited to one (Ia) or both (Ib) of the ovaries and capsule is intact. At stage Ic the tumor is limited to the ovaries but capsule is interrupted and/or tumor on ovarian surface. At stage II, malignancy is present on one (IIa) or both (IIb) of the ovaries, with pelvic extension and ascites. At stage III, microscopic peritoneal implants are present on the outside of the pelvis or is limited to the pelvis with extension to the small bowel or omentum. Stage IV includes distant metastases to the liver or outside the peritoneal cavity. Early stage tumors (Stage I) are generally associated with a relatively favorable outcome, with an overall five-year survival as high as 70-90%. However, the overall five-year survival rate of ovarian cancer is approximately 45 % (1996-2003) in the US (Jemdal A et al (2008) CA Cancer J Clin 58, 71-96) as approximately 70% of the patients receive their diagnosis at an advanced stage (Stage II-IV), thus distant metastases are common.

Ovarian cancer has been named the "silent killer" as symptoms are few and non-specific. Symptoms include fatigue, indigestion, back pain, pain with intercourse, constipation and menstrual irregularities. As these symptoms also commonly are found in women in the general population who do not have ovarian cancer, they are not so useful in identifying ovarian cancer. As a consequence, ovarian cancer is difficult to diagnose until it spreads and advances to later stages.

A conclusive diagnosis requires a surgical procedure, i.e. an open procedure or keyhole surgery where suspicious areas are removed and sent for microscopic analysis. Also fluid from the abdominal cavity may be analyzed for cancerous cells.

Ovarian epithelial cancers comprise of a heterogeneous group of neoplasms including serous-, mucinous- and endometrioid adenocarcinoma as well as clear cell-, transitional-, squamous-, undifferentiated- and mixed carcinoma. The by far most common type is serous adenocarcinoma. This subgroup can be divided into high-grade serous carcinoma and low-grade serous carcinoma with two distinct tumor types with a different underlying pathogenesis. Low-grade serous carcinoma is less aggressive than the high-grade. Clear cell and endometrioid are the second and third most common types. Endometrioid carcinoma is sometimes considered as a less aggressive type of EOC. One explanation for this may be that this type of EOC presents symptoms at an earlier stage and thus is diagnosed earlier than other types of EOC.

### Endpoint analysis

Endpoint analysis for trials with adjuvant treatments for cancer gives important information on how the patients respond to a certain therapy. Overall survival (OS) has long been considered the standard primary endpoint. OS takes in to account time to death, irrespective of cause, e.g. if the death is due to cancer or not. Loss to follow-up is censored and regional recurrence, distant metastases, second primary colorectal cancers and second other primary cancers are ignored.

Today, an increasing number of effective treatments available for many types of cancer have resulted in the need for surrogate endpoints to allow for a better evaluation of the effect of adjuvant treatments. Partly due to the long follow-up period required to demonstrate that adjuvant treatments improve OS, the endpoint is often complemented with other clinical endpoints that gives an earlier indication on how successful the treatment is.

In the present disclosure, the inventors show that the level of expression of a particular protein (the proposed biomarker) significantly correlates with prognoses. For these observations, primarily two surrogate endpoints are used, namely recurrence-free survival (RFS) and ovarian cancer-specific survival (OCSS). Analysis of RFS includes time to any event related to the same cancer, i.e., all cancer recurrences and deaths from the same cancer are events. Also distant, local and regional metastases as well as ovarian cancer specific death are considered. On the other hand, second primary same cancers and other primary cancers are ignored. Deaths from other cancers, non-cancer-related deaths, treatment-related deaths, and loss to follow-up are censored observations. Ovarian cancer-specific survival includes time to death caused by ovarain cancer due to the original tumor. Both endpoints are relevant, since similarities or differences may reflect different tumor biological behaviors. Biomarkers associated with a locally aggressive behavior may for instance have greater impact on RFS than OCSS, while biomarkers associated with the development of distant metastases may be reflected in both RFS and OCSS.

### Brief description

It is an object of some aspects of the present disclosure to provide a method for establishing an ovarian cancer prognosis.

Further, it is an object of some aspects of the present disclosure to provide methods and means for obtaining ovarian cancer treatment-related information, and, in some aspects, perform treatment.

Also, it is an object of some other aspects of the present disclosure to provide a kit for performing one or both of the above methods and other means useful for obtaining prognostic or treatment-related information.

The following is a non-limiting and itemized listing of embodiments of the present disclosure, presented for the purpose of providing various features and combinations provided by the invention in certain of its aspects.

### ITEMS

1. Method for determining whether a mammalian subject having an ovarian cancer belongs to a first or a second group, wherein the prognosis of subjects of the first group is better than the prognosis of subjects of the second group, comprising the steps of:
   a) evaluating the amount of HMGCR protein in at least part of a sample earlier obtained from the subject and determining a sample value corresponding to said evaluated amount;
   b) comparing said sample value with a predetermined reference value;
      and
      if said sample value is higher than said reference value,
   c1) concluding that the subject belongs to the first group; and
      if said sample value is lower than or equal to said reference value,
   c2) concluding that the subject belongs to the second group.
2. Method for determining a prognosis for a mammalian subject having an ovarian cancer, comprising the steps of:
   a) evaluating the amount of HMGCR protein present in at least part of a sample earlier obtained from the subject and determining a sample value corresponding to said evaluated amount;
   b) comparing the sample value obtained in step a) with a reference value associated with a reference prognosis; and,
      if said sample value is higher than said reference value,
   c1) concluding that the prognosis for said subject is better than said
      reference prognosis; or
      if said sample value is lower than or equal to said reference value,
   c2) concluding that the prognosis for said subject is worse than or equal to said reference prognosis.
3. Method for determining whether a subject having an ovarian cancer is not in need of a treatment with an ovarian cancer treatment regimen, comprising the steps of:
   a) evaluating the amount of HMGCR protein present in at least part of a sample earlier obtained from the subject, and determining a sample value corresponding to said evaluated amount;
   b) comparing the sample value obtained in step a) with a reference value; and,
      if said sample value is higher than said reference value,
   c) concluding that said subject is not in need of the treatment with the ovarian cancer treatment regimen.
4. Non-treatment strategy method for a subject having an ovarian cancer, comprising:
   a) evaluating the amount of HMGCR protein present in at least part of a sample earlier obtained from the subject, and determining a sample value corresponding to said evaluated amount;
   b) comparing the sample value obtained in step a) with a reference value; and,
      if said sample value is higher than said reference value,
   c) refraining from treating said subject with an ovarian cancer treatment regimen.
5. Method of treatment of a subject having an ovarian cancer, comprising:
   a) evaluating the amount of HMGCR protein present in at least part of a sample from the subject and determining a sample value corresponding to said evaluated amount;
   b) comparing the sample value obtained in step a) with a reference value; and, if said sample value is equal to or lower than said reference value,
   c) treating said subject with an ovarian cancer treatment regimen.
6. Method according to item 1 or 2, wherein said prognosis is a probability of survival, such as overall survival, recurrence free survival or ovarian cancer specific survival.
7. Method according to item 6, wherein the probability of survival is a probability of five-year, ten-year or 15-year survival.
8. Method according to any one of items 3-5, wherein said ovarian cancer treatment regimen comprises adjuvant and/or neo-adjuvant therapy.
9. Method according to any one of items 3-5 and 8, wherein said ovarian cancer treatment regimen comprises chemotherapy.
10. Method according to item 9, wherein said chemotherapy comprises treatment with one or more platinum-based drugs, such as carboplatin, paraplatin and cisplatin.
11. Method according to item 9 or 10, wherein said chemotherapy comprises treatment with one or more drugs selected from taxanes, such as paclitaxel and docetaxel, gemcitabine, topoisomerase inhibitors, such as topotecan, and anthracyclins, such as doxorubicin.
12. Method according to any one of items 9-11, wherein said chemotherapy comprises treatment with a combination of carboplatin and paclitaxel.
13. Method according to any one of items 3-5 and 8-12, wherein said ovarian cancer treatment regimen comprises radiation therapy.
14. Method according to any one of the preceding items, wherein ovarian cancer is poorly or moderately differentiated.
15. Method according to any one of the preceding items, wherein said ovarian cancer is in stage 1 according to the Federation of Gynecology and Obstetrics (FIGO) classification.
16. Method according to any one of items 1-14, wherein said ovarian cancer is in stage 1, 2 or 3 according to the Federation of Gynecology and Obstetrics (FIGO) classification.
17. Method according to any one of items 1-14, wherein said ovarian cancer is in stage 2 or 3 according to the Federation of Gynecology and Obstetrics (FIGO) classification.
18. Method according to any one of the preceding items, wherein said ovarian cancer is selected from serous and endometroid ovarian cancer.
19. Method according to any one of the preceding items, wherein said sample is a body fluid sample, stool sample or cytology sample.
20.Method according to item 19, wherein said body fluid sample is selected from the group consisting of blood, plasma, serum, cerebral fluid, urine and exudate.
21. Method according to any one of the preceding items, wherein said sample comprises cells, such as tumor cells, from said subject.
22. Method according to any one of items 1-18, wherein said sample is an ovarian tissue sample, such as an ovarian tumor tissue sample.
23. Method according to any one of items 21-22, wherein the evaluation of step a) is limited to the cytoplasms of cells of said sample.
24. Method according to item 23, wherein the evaluation of step a) is limited to the cytoplasms of tumor cells of said sample.
25. Method according to any one of the preceding items, wherein said subject is a human.
26. Method according to any one of the preceding items, wherein said reference value is a cytoplasmic fraction, a cytoplasmic intensity or a function of a cytoplasmic fraction and a cytoplasmic intensity.
27. Method according to item 26, wherein said reference value is a cytoplasmic intensity.
28. Method according to item 27, wherein said reference value is selected from an absent, weak, weak/moderate and moderate cytoplasmic intensity.
29. Method according to any one of items 1-25, wherein said reference value is an autoscore, such as an autoscore of 20-50.
30. Method according to any one of the preceding items, wherein the amino acid sequence of the HMGCR protein comprises a sequence selected from:
   i) SEQ ID NO:1; and
   ii) a sequence which is at least 85 % identical to SEQ ID NO:1.
31. Method according to any one of the preceding items, wherein the amino acid sequence of the HMGCR protein comprises or consists of a sequence selected from:
   i) SEQ ID NO:2; and
   ii) a sequence which is at least 85 % identical to SEQ ID NO:2.
32. Method according to any one of the preceding items, wherein step a) comprises:
   al) applying to said sample a quantifiable affinity ligand capable of selective interaction with the HMGCR protein to be evaluated, said application being performed under conditions that enable binding of the affinity ligand to HMGCR protein present in the sample; and
   all) quantifying the affinity ligand bound to said sample to evaluate said amount.
33. Method according to any one of items 1-31, wherein step a) comprises:
   a1) applying to said sample a quantifiable affinity ligand capable of selective interaction with the HMGCR protein to be quantified, said application being performed under conditions that enable binding of the affinity ligand to HMGCR protein present in the sample;
   a2) removing non-bound affinity ligand; and
   a3) quantifying affinity ligand remaining in association with the sample to evaluate said amount.
34. Method according to item 32 or 33, wherein said quantifiable affinity ligand is selected from the group consisting of antibodies, fragments thereof and derivatives thereof.
35. Method according to item 32 or 33, wherein said quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
36. Method according to item 32 or 33, wherein said quantifiable affinity ligand is an oligonucleotide molecule.
37. Method according to any one of items 32-36, wherein said quantifiable affinity ligand is capable of selective interaction with an HMGCR protein whose amino acid sequence consists of a sequence selected from SEQ ID NO:1 and SEQ ID NO:3.
38. Method according to any one of items 32-37, wherein said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
39. Method according to any one of items 32-38, wherein said quantifiable affinity ligand is detected using a secondary affinity ligand capable of recognizing said quantifiable affinity ligand.
40. Method according to item 39, wherein said secondary affinity ligand is capable of recognizing said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
41. Kit for carrying out a method according to any one of the preceding items, which comprises
   a) a quantifiable affinity ligand capable of selective interaction with an HMGCR protein; and
   b) reagents necessary for quantifying the amount of said quantifiable affinity ligand.
42. Kit according to item 41, in which said quantifiable affinity ligand is selected from the group consisting of antibodies, fragments thereof and derivatives thereof.
43. Kit according to item 41, in which said quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
44. Kit according to item 41, in which said quantifiable affinity ligand is an oligonucleotide molecule.
45. Kit according to any one of item 41-44, in which said quantifiable affinity ligand is capable of selective interaction with an HMGCR protein comprising, or consisting of, a sequence selected from:
   i) SEQ ID NO:2; and
   ii) a sequence which is at least 85 % identical to SEQ ID NO:2.
46. Kit according to any one of items 41-45, in which said quantifiable affinity ligand is capable of selective interaction with an HMGCR protein comprising, or consisting of, a sequence selected from:
   i) SEQ ID NO:1; and
   ii) a sequence which is at least 85 % identical to SEQ ID NO:1.
47. Kit according to any one of items 41-46, in which said quantifiable affinity ligand is capable of selective interaction with an HMGCR protein comprising, or consisting of, a sequence selected from:
   i) SEQ ID NO:3; and
   ii) a sequence which is at least 85 % identical to SEQ ID NO:3.
48. Kit according to any one of items 41-47, in which said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
49. Kit according to any one of items 41-48, in which said reagents necessary for quantifying said amount of said quantifiable affinity ligand comprise a secondary affinity ligand capable of recognizing said quantifiable affinity ligand.
50. Kit according to item 49, in which said secondary affinity ligand comprises a label selected from the group consisting of fluorescent dyes or metals, chromophoric dyes, chemiluminescent compounds and bioluminescent proteins, enzymes, radioisotopes, particles and quantum dots.
51. Kit according to any one of items 41-50, further comprising at least one reference sample for provision of a reference value.
52. Kit according to item 51, in which at least one reference sample is a tissue sample comprising no detectable HMGCR protein.
53. Kit according to item 51 or 52, in which at least one reference sample comprises HMGCR protein.
54. Kit according to any one of items 51-53, in which at least one reference sample comprises an amount of HMGCR protein corresponding to a weak, weak/moderate or moderate cytoplasmic intensity.
55. Kit according to any one of items 51-53, in which at least one reference sample comprises an amount of HMGCR protein corresponding to an autoscore of 20-50.
56. Kit according to any one of items 51-55, in which at least one reference sample comprises an amount of HMGCR protein corresponding to a value being higher than said reference value.
57. Kit according to item 56, in which the value being higher than said reference value is a strong cytoplasmic intensity.
58. Kit according to any one of items 51-57 comprising:
   a first reference sample comprising an amount of HMGCR protein corresponding to a value (positive reference value) being higher than a reference value; and
   a second reference sample comprising an amount of HMGCR protein corresponding to a value (negative reference value) being lower than or equal to said reference value.
59. Kit according to any one of items 51-58, in which said reference sample(s) is/are tissue sample(s).
60. Use *in vitro* of an HMGCR protein as a prognostic marker.
61. Use according to item 60, wherein said prognostic marker is a prognostic marker for a cancer.
62. Use according to item 61, wherein said cancer is an ovarian cancer.
63. Use according to item 62, wherein said protein is provided in a biological sample from a subject having an ovarian cancer.
64. Use according to any one of items 60-63, wherein said protein is provided in an ovarian tumor tissue sample.
65. Use *in vitro* of an HMGCR protein, or an antigenically active fragment thereof, for the production, selection or purification of a prognostic agent for establishing a prognosis for a mammalian subject having an ovarian cancer.
66. Use according to item 65, wherein said prognostic agent is an affinity ligand capable of selective interaction with the HMGCR protein, or an antigenically active fragment thereof.
67. Use according any one of items 65-66, wherein the amino acid sequence of the HMGCR protein or antigenically active fragment thereof comprises a sequence selected from:
   i) SEQ ID NO:1; and
   ii) a sequence which is at least 85 % identical to SEQ ID NO:1.
68. Affinity ligand capable of selective interaction with an HMGCR protein, which is capable of selective interaction with a peptide whose amino acid sequence consists of a sequence SEQ ID NO:1.
69. Affinity ligand according to item 68, which is an antibody or a fragment or a derivative thereof.
70. Antibody or a fragment or a derivative thereof obtainable by a process comprising a step of immunizing an animal with a peptide whose amino acid sequence consists of a sequence SEQ ID NO:1.
71. Affinity ligand capable of selective interaction with a HMGCR protein for use *in vivo* for establishing a prognosis for a mammalian subject having an ovarian cancer.
72. Use *in vitro* of an affinity ligand capable of selective interaction with a HMGCR protein as a prognostic agent.
73. Use according to item 72 as a prognostic agent for ovarian cancer.
74. Use of an affinity ligand capable of selective interaction with a HMGCR protein in the manufacture of a prognostic agent for *in vivo* establishment of a prognosis for a mammalian subject having an ovarian cancer.
75.Use according to any one of items 72-74, wherein the affinity ligand is an antibody.
76.Use according to any one of items 72-74, wherein the affinity ligand is an affinity ligand according to any one of items 68-70.

### Brief description of the figures

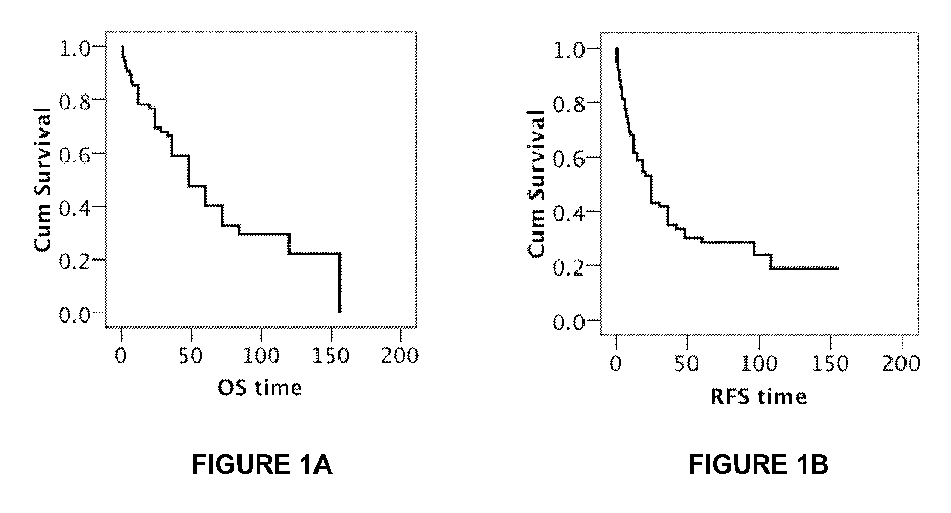

Figure 1 shows the results of a survival analysis of 71 subjects diagnosed with epithelial ovarian cancer (EOC). Figure 1 a shows overall survival (OS) in all patients, estimated five-year survival was 40% for all patients in this cohort. Figure 1b shows recurrence free survival (RFS) in all patients, estimated five-year survival was 29% for all patients in this cohort.

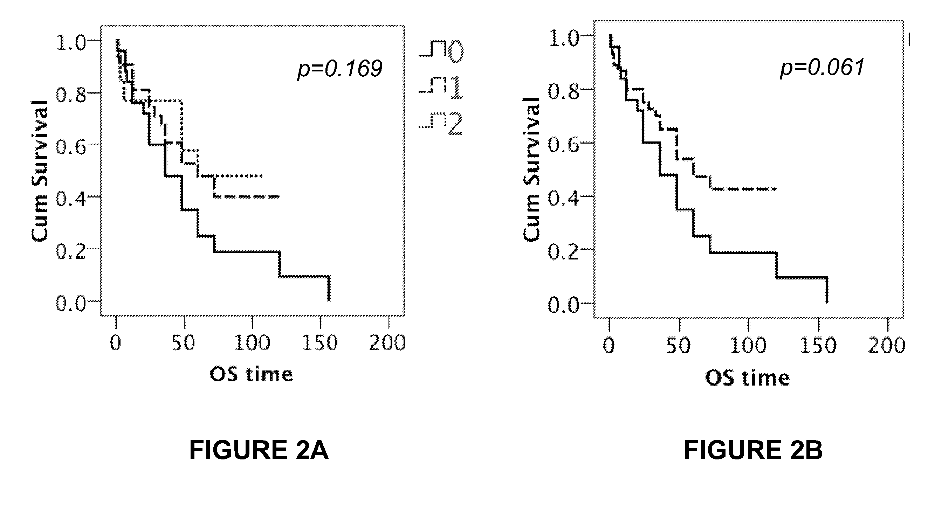

Figure 2 shows the results of an OS analysis for all 71 subjects based on cytoplasmic intensity (CI) levels of HMGCR. In figure 2A subjects were split into three groups based on Cl, i.e. 0 = absent, 1 = weak/moderate and 2 = strong. In Figure 2B HMGCR expression were dichotomized into high and low categories. A solid line represents a low HMGCR level (CI = 0), and a dotted line represents a high HMGCR level (CI > 0).

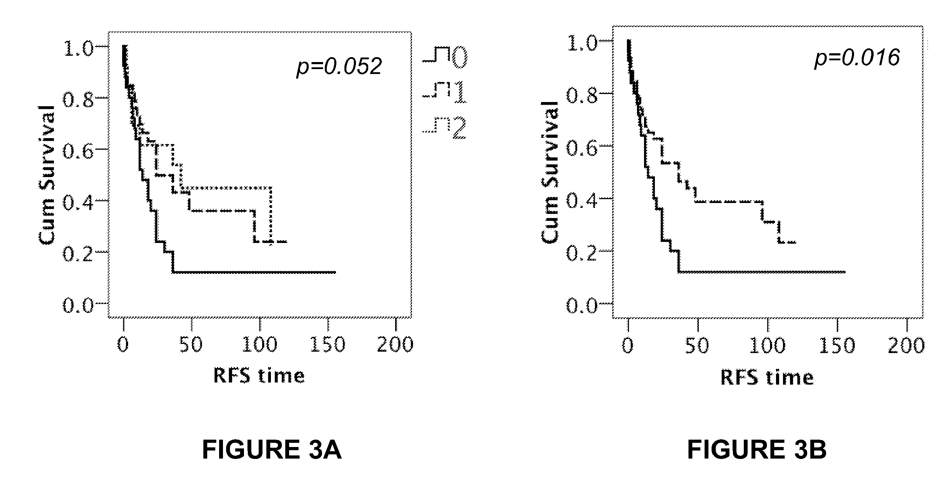

Figure 3 shows the results of a RFS analysis for all 71 subjects based on cytoplasmic intensity (CI) levels of HMGCR. In figure 3A subjects were split into three groups based on CI, i.e. 0 = absent, 1 = weak/moderate and 2 = strong. In Figure 3B HMGCR expression were dichotomized into high and low categories. A solid line represents a low HMGCR level (CI = 0), and a dotted line represents a high HMGCR level (CI > 0).

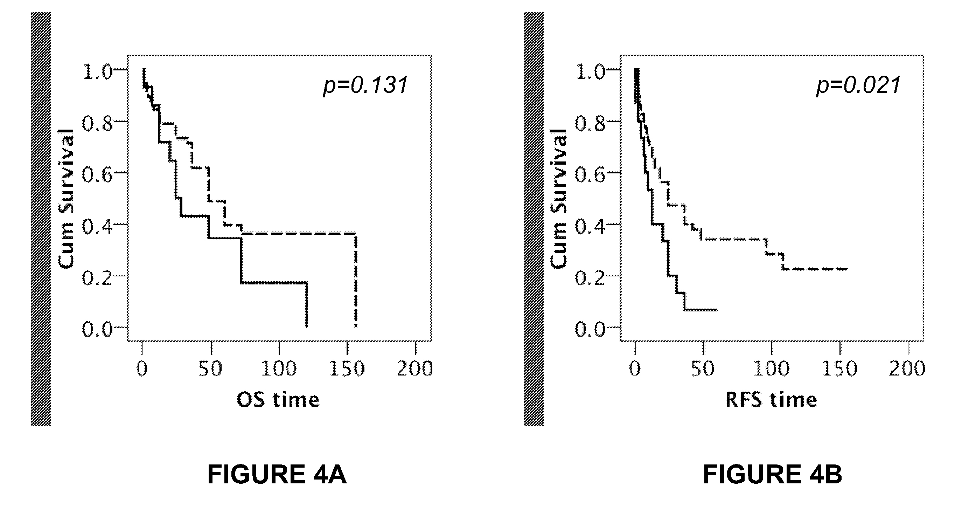

Figure 4 shows the results of survival analysis of all 71 patients when applying automatic analysis. Tissue cores were dichotomized by high and low HMGCR expression. A solid line represents a low HMGCR level (AS < 35), and a dotted line represents a high HMGCR level (AS > 35). Figure 4A shows OS. Figure 4B shows RFS.

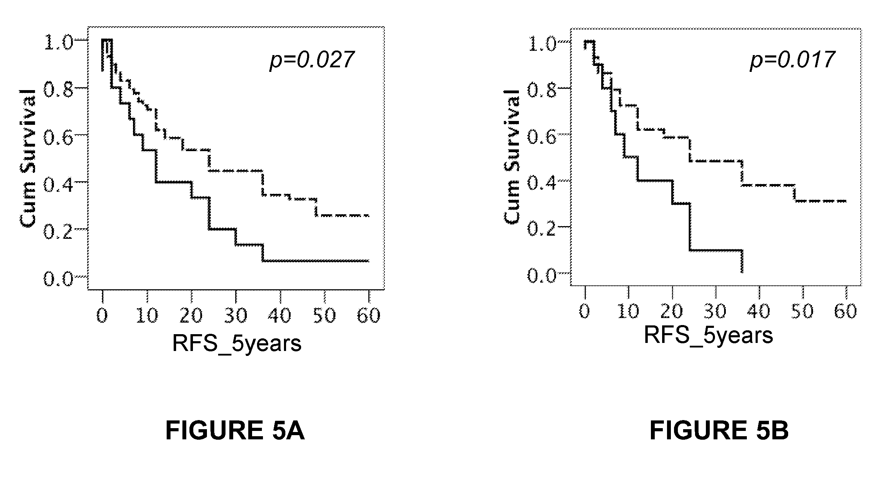

Figure 5 shows the results of a five-year survival analysis of all 71 patients when applying automatic analysis. Tissue cores were dichotomized by high and low HMGCR expression. A solid line represents a low HMGCR level (AS < 35), and a dotted line represents a high HMGCR level (AS > 35). Figure 5A shows OS. Figure 5B shows RFS.

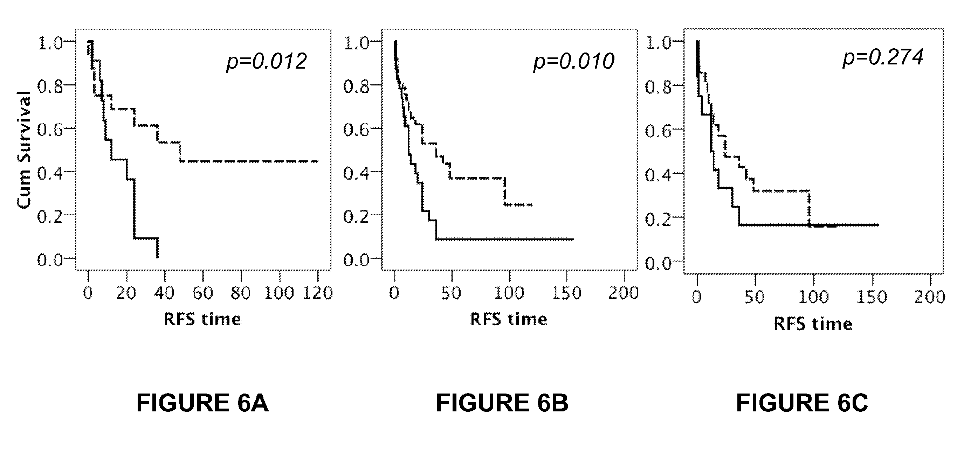

Figure 6 shows the results of RFS analysis for patients diagnosed with grade 2, grade 2 or 3, and grade 3 EOC based on cytoplasmic intensity (CI) levels of HMGCR. Tissue cores were dichotomized by high and low HMGCR expression. A solid line represents a low HMGCR level (CI = 0), and a dotted line represents a high HMGCR level (CI > 0). Figure 6A shows patients with grade 2 EOC. Figure 6B shows patients with grade 2 or grade 3 EOC. Figure 6c shows patients with grade 3 EOC.

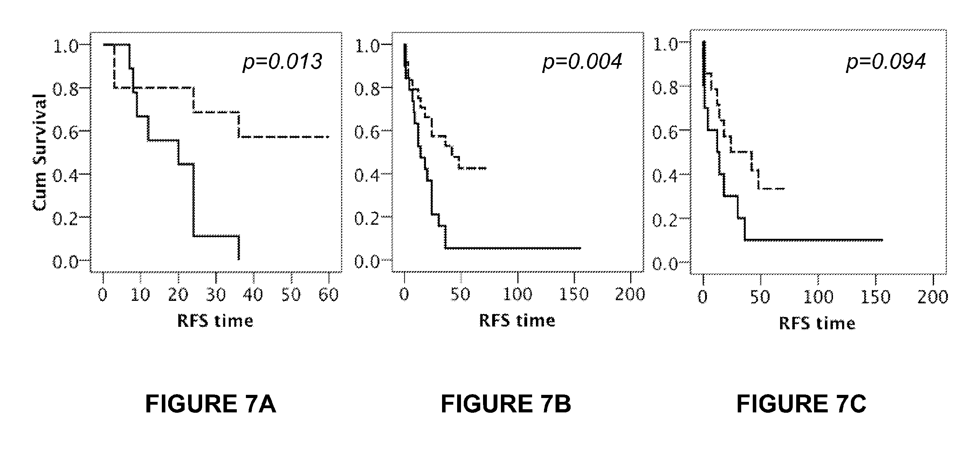

Figure 7 shows the results of RFS analysis for patients diagnosed with grade 2, grade 2 or 3, and grade 3 serous EOC based on cytoplasmic intensity (CI) levels of HMGCR. Tissue cores were dichotomized by high and low HMGCR expression. A solid line represents a low HMGCR level (CI = 0), and a dotted line represents a high HMGCR level (CI > 0). Figure 7A shows patients with grade 2 serous EOC. Figure 7B shows patients with grade 2 or grade 3 serous EOC. Figure 7C shows patients with grade 3 serous EOC.

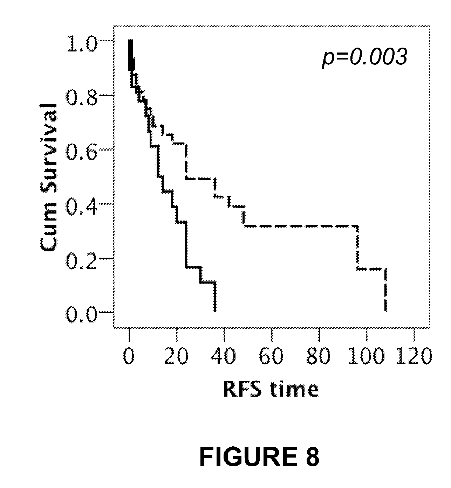

Figure 8 shows the results of a RFS analysis for patients diagnosed with stage 3 EOC, based on cytoplasmic intensity (CI) levels of HMGCR. Tissue cores were dichotomized by high and low HMGCR expression. A solid line represents a low HMGCR level (CI = 0), and a dotted line represents a high HMGCR level (CI > 0).

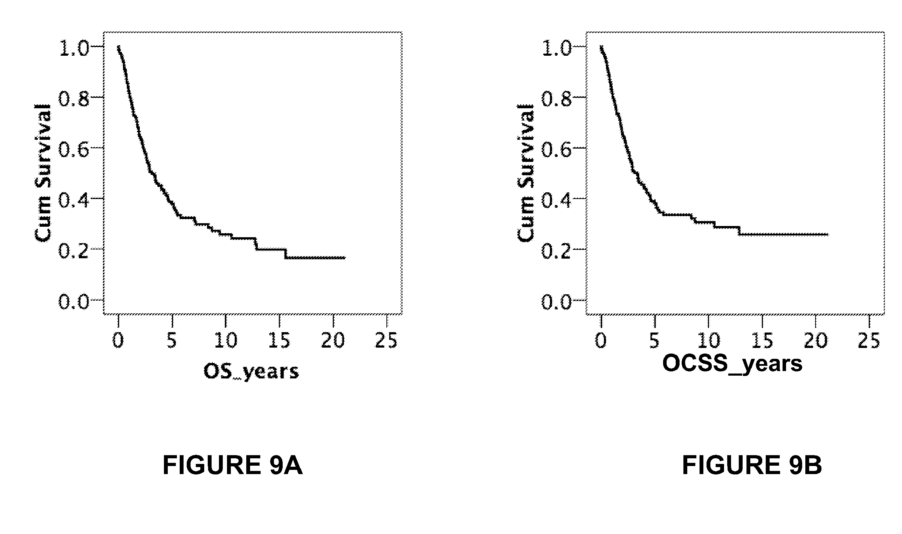

Figure 9 shows the results of a survival analysis of 154 subjects diagnosed with epithelial ovarian cancer (EOC). Figure 9A shows overall survival (OS) in all patients, estimated five-year survival is 32% for all patients in this cohort. Figure 9B shows ovarian cancer specific survival (OCSS) in all patients, estimated five-year survival is 29% for all patients in this cohort.

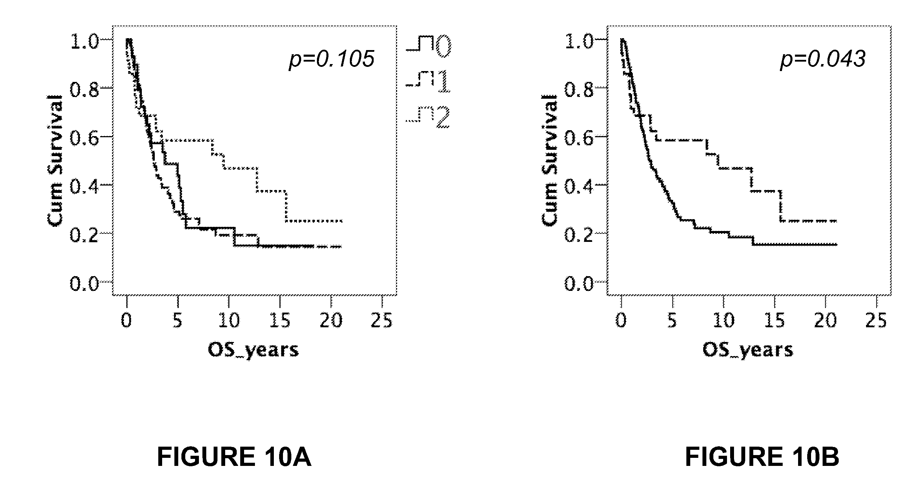

Figure 10 shows the results of OS analysis for all 154 subjects based on cytoplasmic intensity (CI) levels of HMGCR. In figure 10A subjects were split into three groups based on CI, i.e. 0 = absent, 1 = weak/moderate and 2 = strong. In Figure 10B HMGCR expression were dichotomized into high and low categories. A solid line represents a low HMGCR level (CI < 2), and a dotted line represents a high HMGCR level (CI = 2).

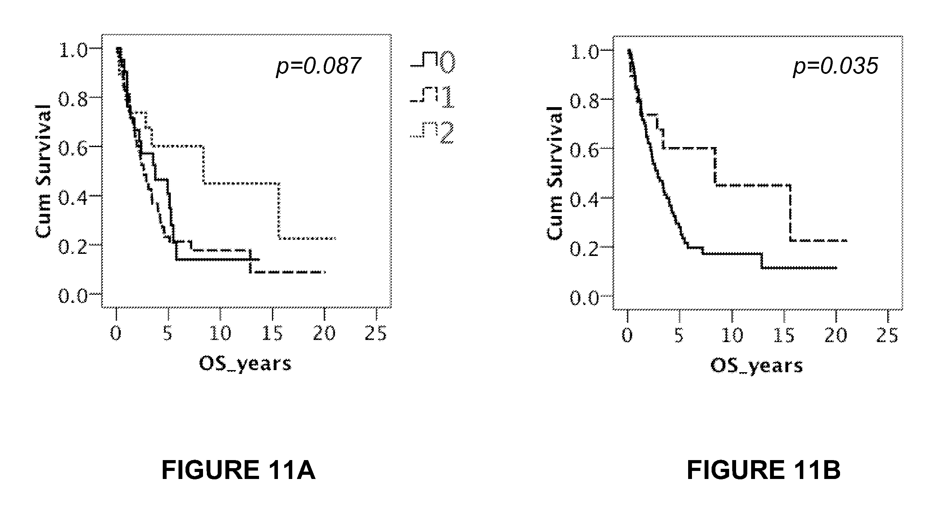

Figure 11 shows the results of OS analysis for patients diagnosed with stage 2 or 3 EOC based on cytoplasmic intensity (CI) levels of HMGCR. In figure 11A subjects were split into three groups based on CI, i.e. 0 = absent, 1 = weak/moderate and 2 = strong. In Figure 11B HMGCR expression were dichotomized into high and low categories. A solid line represents a low HMGCR level (CI < 2), and a dotted line represents a high HMGCR level (CI = 2).

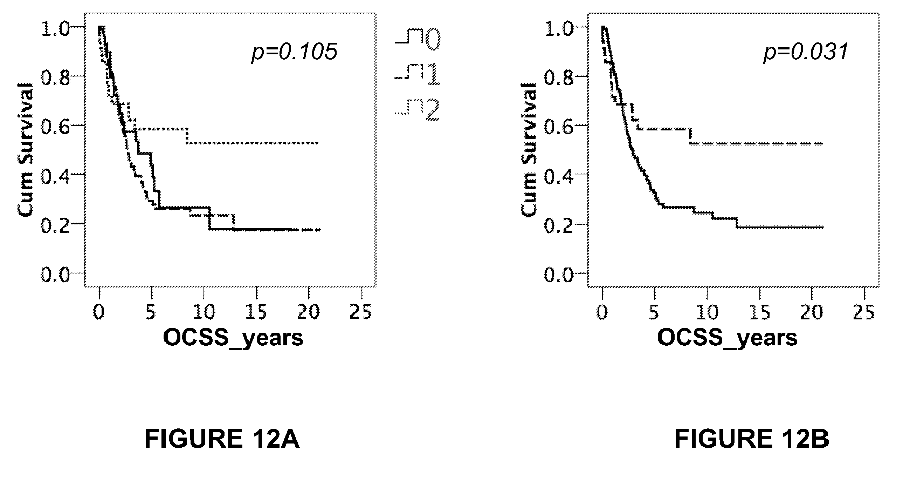

Figure 12 shows the results of an OCSS analysis for all 154 subjects based on cytoplasmic intensity (CI) levels of HMGCR. In figure 12A subjects were split into three groups based on CI, i.e. 0 = absent, 1 = weak/moderate and 2 = strong. In Figure 12B HMGCR expression were dichotomized into high and low categories. A solid line represents a low HMGCR level (CI < 2), and a dotted line represents a high HMGCR level (CI = 2).

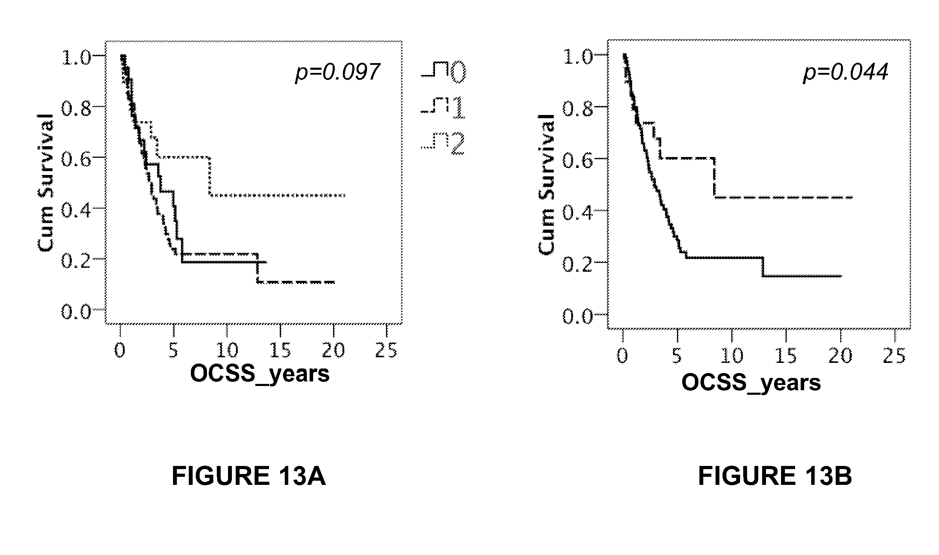

Figure 13 shows the results of OCSS analysis for patients diagnosed with stage 2 or 3 EOC based on cytoplasmic intensity (CI) levels of HMGCR. In figure 13A subjects were split into three groups based on CI, i.e. 0 = absent, 1 = weak/moderate and 2 = strong. In Figure 13B HMGCR expression were dichotomized into high and low categories. A solid line represents a low HMGCR level (CI < 2), and a dotted line represents a high HMGCR level (CI = 2).

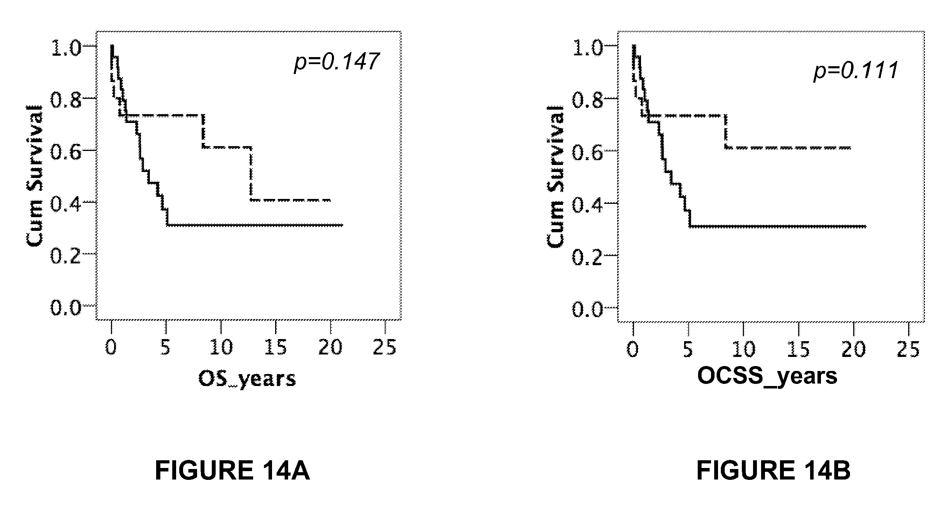

Figure 14 shows the results of survival analysis of patients diagnosed with grade 2 EOC based on cytoplasmic intensity (CI) levels of HMGCR. Tissue cores were dichotomized by high and low HMGCR expression. A solid line represents a low HMGCR level (CI < 2), and a dotted line represents a high HMGCR level (CI = 2). Figure 14a shows OS. Figure 14b shows OCSS.

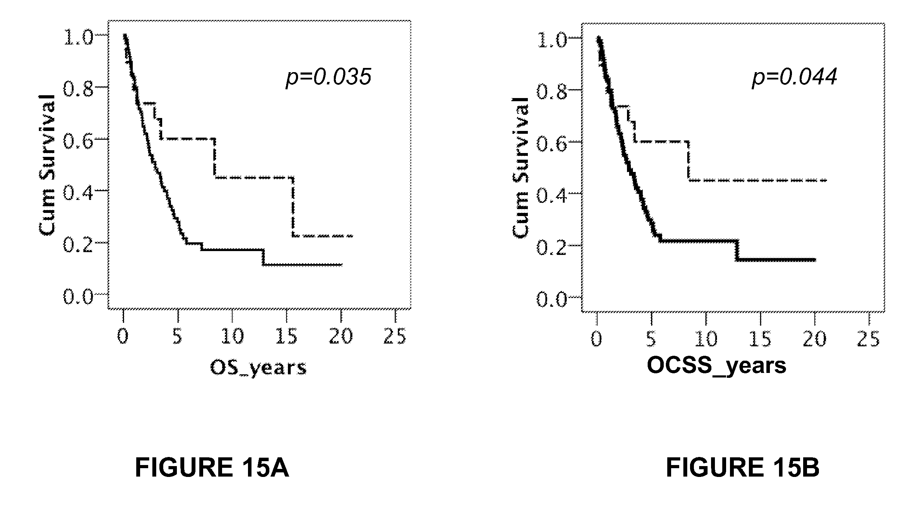

Figure 15 shows the results of survival analysis of patients diagnosed with stage 2 or 3 EOC based on cytoplasmic intensity (CI) levels of HMGCR. Tissue cores were dichotomized by high and low HMGCR expression. A solid line represents a low HMGCR level (CI < 2), and a dotted line represents a high HMGCR level (CI = 2). Figure 15a shows OS. Figure 15b shows OCSS.

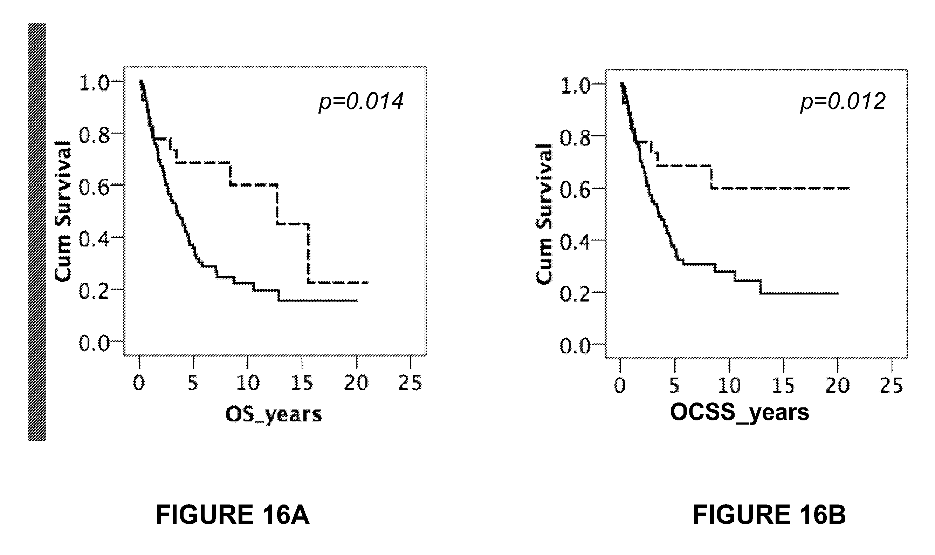

Figure 16 shows the results of survival analysis of patients diagnosed with stage < 4 EOC based on cytoplasmic intensity (CI) levels of HMGCR. Tissue cores were dichotomized by high and low HMGCR expression. A solid line represents a low HMGCR level (CI < 2), and a dotted line represents a high HMGCR level (CI = 2). Figure 16a shows OS. Figure 16b shows OCSS.

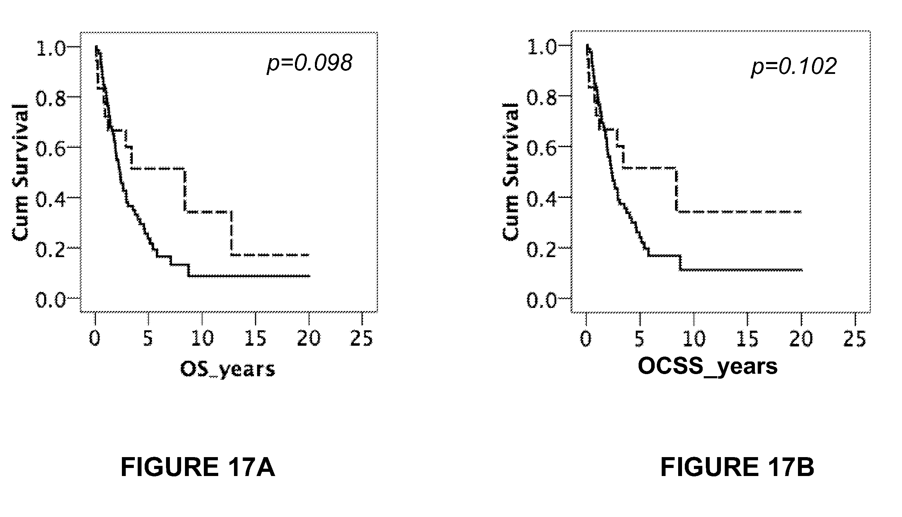

Figure 17 shows the results of survival analysis of patients diagnosed with serous EOC based on cytoplasmic intensity (CI) levels of HMGCR. Tissue cores were dichotomized by high and low HMGCR expression. A solid line represents a low HMGCR level (CI < 2), and a dotted line represents a high HMGCR level (CI = 2). Figure 17a shows OS. Figure 17b shows OCSS.

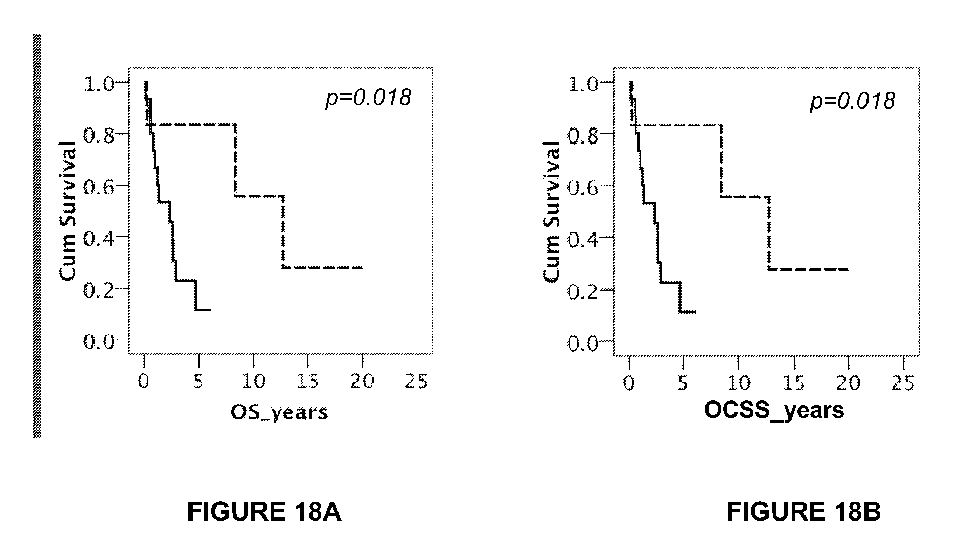

Figure 18 shows the results of survival analysis of patients diagnosed with serous grade 2 EOC based on cytoplasmic intensity (CI) levels of HMGCR. Tissue cores were dichotomized by high and low HMGCR expression. A solid line represents a low HMGCR level (CI < 2), and a dotted line represents a high HMGCR level (CI = 2). Figure 18a shows OS. Figure 18b shows OCSS.

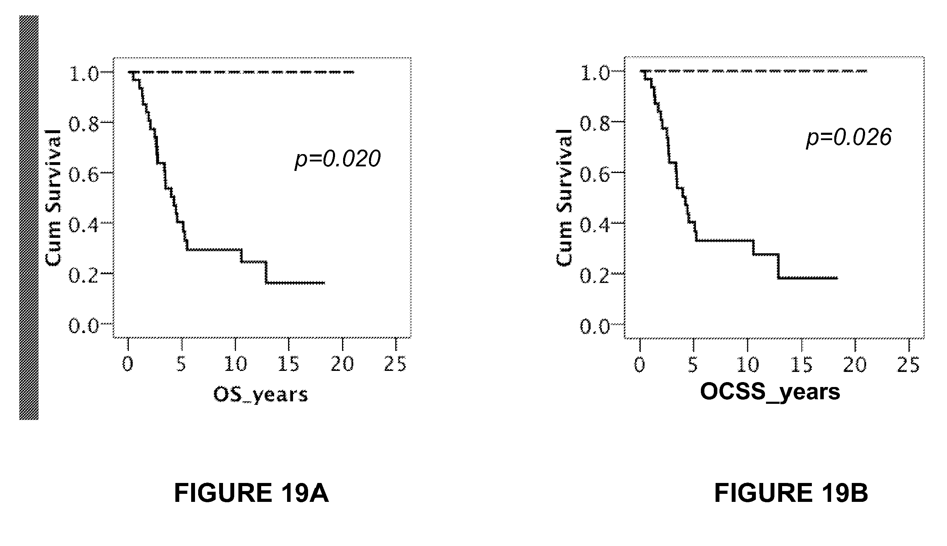

Figure 19 shows the results of survival analysis of patients diagnosed with endometrioid OC based on cytoplasmic intensity (CI) levels of HMGCR. Tissue cores were dichotomized by high and low HMGCR expression. A solid line represents a low HMGCR level (CI < 2), and a dotted line represents a high HMGCR level (CI = 2). Figure 19a shows OS. Figure 19b shows OCSS.

### Detailed description

As a first aspect of the present disclosure, there is thus provided a method for determining whether a mammalian subject having an ovarian cancer belongs to a first or a second group, wherein the prognosis of subjects of the first group is better than the prognosis of subjects of the second group, comprising the steps of:
a) evaluating the amount of HMGCR protein in at least part of a sample earlier obtained from the subject and determining a sample value corresponding to said evaluated amount;
b) comparing said sample value with a predetermined reference value;
   and
   if said sample value is higher than said reference value,
c1) concluding that the subject belongs to the first group; and
   if said sample value is lower than or equal to said reference value,
c2) concluding that the subject belongs to the second group.

The present invention based on 3-Hydroxy-3-Methylglutaryl-Coenzyme A reductase (HMGCR) protein levels as an ovarian cancer status indicator has a number of benefits. As well known by the person skilled in the art, a prognosis may be important for various reasons. Frequently, the prognosis for an ovarian cancer subject reflects the aggressiveness of the cancer. In general, identification of the aggressiveness of an ovarian cancer is of vital importance as it helps a physician selecting an appropriate treatment strategy. For example, if a particularly aggressive form of a cancer is identified, a painful or in any other sense unpleasant treatment which normally is avoided may anyway be considered. Further, if less aggressive forms can be identified, over-treatment may be avoided. As a further example, the HMGCR protein, as a marker for which a certain level of expression is correlated with a certain pattern of disease progression, has a great potential for example in a panel for making predictions or prognoses or for the selection of a treatment regimen.

In the method of the first aspect, it is determined whether an ovarian cancer subject belongs to a first or a second group, wherein subjects of the first group generally have a better prognosis than subjects of the second group. The division of ovarian cancer subjects into the two groups is determined by comparing samples values from the subjects with a reference value. In the present disclosure it is shown that various reference values may be employed to discriminate between subjects that generally survived for a comparatively long period (represented by the upper curve) and subjects that generally survived for a comparatively short period (represented by the lower curve). The reference value is thus the determinant for the size of the respective groups; the higher the reference value, the fewer the subjects in the first group and the lower the likelihood that a tested subject belongs to the first group. As the prognosis generally improves with the sample value, a high reference value may in some instances be selected to identify subjects with a particularly good prognosis. Guided by the present disclosure, the person skilled in the art may select relevant reference values without undue burden. This is further discussed below.

The first and the second group may consist exclusively of subjects having ovarian cancers of the same stage (see figures 8, 11, 13, 15 and 16), differentiation grade (see figures 6, 7, 14 and 18) and/or type (see figures 7, 17, 18 and 19) as the tested subject. Further, the groups may consist only of subjects having the same or similar age, race, menopausal status, genetic characteristics or medical status or history.

Consequently, a physician may use the method according to the first aspect to obtain additional information regarding the prognosis of an ovarian cancer subject, which in turn may help him to select the most appropriate treatment regimen. For example, a subject shown to belong to the first group using the method of the first aspect may be given a less severe treatment than what would otherwise have been considered, and vice versa.

The prognosis of the tested subject may also be determined relative a reference prognosis. Accordingly, as a first configuration of the first aspect, there is provided a method for determining a prognosis for a mammalian subject having an ovarian cancer, comprising the steps of:
a) evaluating the amount of HMGCR protein present in at least part of a sample earlier obtained from the subject and determining a sample value corresponding to said evaluated amount;
b) comparing the sample value obtained in step a) with a reference value associated with a reference prognosis; and, if said sample value is higher than said reference value,
c1) concluding that the prognosis for said subject is better than said reference prognosis; and/or
   if said sample value is lower than or equal to said reference value,
c2) concluding that the prognosis for said subject is worse than or equal to said reference prognosis.

However closely related and covered by the same concept, c1) and c2) provide two alternative conclusions.

Similarly and as a second configuration of the first aspect, there is provided a method for determining whether a prognosis for a mammalian subject having an ovarian cancer is better than a reference prognosis, comprising the steps of:
a) evaluating the amount of HMGCR protein present in at least part of a sample earlier obtained from the subject, and determining a sample value corresponding to said evaluated amount;
b) comparing the sample value obtained in step a) with a reference value associated with said reference prognosis; and,
   if said sample value is higher than said reference value,
c) concluding that the prognosis for said subject is better than said reference prognosis.

It follows that as a third configuration of the first aspect, there is provided a method for determining whether a prognosis for a mammalian subject having an ovarian cancer is worse than or equal to a reference prognosis, comprising the steps of:
a) evaluating the amount of HMGCR protein present in at least part of a sample earlier obtained from the subject, and determining a sample value corresponding to said evaluated amount;
b) comparing the sample value obtained in step a) with a reference value associated with said reference prognosis; and,
   if said sample value is higher lower than or equal to said reference value,
c) concluding that the prognosis for said subject is worse than or equal to said reference prognosis.

The inventive concept of the present disclosure may also form the basis for a decision to refrain from a certain treatment regimen.

For example, as shown in the attached figures 2-8 and 10-19, the prognoses for subjects showing high HMGCR protein levels are generally better than those for subjects showing low HMGCR protein levels. Provided with the teachings of the present disclosure, a physician may consider the prognosis of an HMGCR protein high subject as being so favorable that one adjuvant treatment regimen is avoided and another less aggressive adjuvant treatment regimen is selected instead. For example, a monotherapy may be selected instead of a combination therapy or a therapeutic agent may be given in a lower dose. Further, the decision may be to refrain from any adjuvant therapy. Refraining from any adjuvant therapy may be particularly relevant when said ovarian cancer is an endometroid ovarian cancer as the prognoses for HMGCR high subjects having such cancer appears to be particularly good (see fig 19).

In conclusion, the present disclosure may relieve subjects from over-treatment.

Thus, as a fourth configuration of the first aspect, there is provided a method for determining whether a subject having an ovarian cancer is not in need of a treatment with an ovarian cancer treatment regimen, comprising the steps of:
a) evaluating the amount of HMGCR protein present in at least part of a sample earlier obtained from the subject, and determining a sample value corresponding to said evaluated amount;
b) comparing the sample value obtained in step a) with a reference value; and,
   if said sample value is higher than said reference value,
c) concluding that said subject is not in need of the treatment with the ovarian cancer treatment regimen.

Further, as a fifth configuration of the first aspect, there is provided a non-treatment strategy method for a subject having an ovarian cancer, comprising:
a) evaluating the amount of HMGCR protein present in at least part of a sample earlier obtained from the subject, and determining a sample value corresponding to said evaluated amount;
b) comparing the sample value obtained in step a) with a reference value; and,
   if said sample value is higher than said reference value,
c) refraining from treating said subject with an ovarian cancer treatment regimen.

For example, the refraining of step c) of the fifth configuration may be a refraining from treatment during at least one week from the completion of steps a) - b), such as at least one month from the completion of steps a) - b), such as at least three months from the completion of steps a) - b), such as at least six months from the completion of steps a) - b), such as at least one year from the completion of steps a) - b), such as at least two years from the completion of steps a) - b).

Alternatively, the refraining of step c) may be a refraining from treatment until the next time the method is performed or until recurrence of an ovarian cancer tumor.

As an alternative configuration of the first aspect, there is provided a method for establishing a prognosis for a mammalian subject having an ovarian cancer, comprising the steps of:
a) evaluating the amount of HMGCR protein present in at least part of a sample from the subject, and determining a sample value corresponding to the evaluated amount; and
b) correlating the sample value of step a) to the prognosis for the subject.

In the context of the present disclosure, "establishing a prognosis" refers to establishing a specific prognosis or a prognosis interval.

In an embodiment of the alternative configuration, the sample may be an earlier obtained sample.

The correlating of step b) refers to any way of associating survival data to the obtained sample value so as to establish a prognosis for the subject.

In the present disclosure, different HMGCR protein values (sample values) corresponding to various prognoses are presented. Typically, a low sample value is associated with a poorer prognosis than a high sample value. In the method of the third configuration of the first aspect, the sample value is compared to a reference value, and if the sample value is equal to or lower than the reference value, it is concluded that the prognosis for the subject is equal to, or worse than, a reference prognosis associated with the reference value.

Consequently, the method may be adapted to a reference value. In such case, starting from a sample value which under the circumstances is considered to be relevant, a reference value which is equal to the sample value may be selected. Subsequently, a reference prognosis associated with that reference value may be established. Guided by the present disclosure, the person skilled in the art understands how to establish a reference prognosis which corresponds to a given reference value. For example, the relation between sample values and survival data in a relevant group of cancer patients may be examined in line with what is described in Examples, Section 2, below. The procedure described therein may be adapted to a given reference value. Then, a prognosis corresponding to the given reference value may be selected as the reference prognosis.

Also, the above method may be adapted to a given reference prognosis. In such case, starting from a reference prognosis which under the circumstances is considered to be relevant, for example for selecting an appropriate therapy, a corresponding reference value may be established. Guided by the present disclosure, the person skilled in the art understands how to establish a reference value which corresponds to a given reference prognosis. For example, the relation between sample values and survival data in a group of cancer patients may be examined as in Examples, Section 2, below, but the procedure described therein may be adapted to establish reference values corresponding to a given reference prognosis. For example, different reference values may be tested until one which correlates with the given reference prognosis is found.

The reasoning above applies mutatis mutandis to the first and second configurations of the first aspect.

Accordingly, the reference prognosis of the first aspect may be based on a previously established prognosis, e.g., obtained by an examination of a relevant population of subjects. Such reference population may be selected to match the tested subject's age, sex, race, ovarian cancer stage and/or medical status and history. Further, a prognosis may be adapted to a background risk in the general population, a statistical prognosis/risk or an assumption based on an examination of the subject. Such examination may also comprise the subject's age, sex, race, ovarian cancer stage, menopausal status and/or medical status and history. Thus, a physician may for example adapt the reference prognosis to the subject's ovarian cancer history, the stage of the tumor, the morphology of the tumor, the location of the tumor, the menopausal status, the presence and spread of metastases and/or further cancer characteristics.

In general, when deciding on a suitable treatment strategy for a patient having ovarian cancer, the physician responsible for the treatment may take several parameters into account, such as the result of an immunohistochemical evaluation, patient age, tumor type, stage and grade, hormone receptor status, general condition and medical history, such as ovarian cancer history. To be guided in the decision, the physician may perform a HMGCR protein test, or order a HMGCR protein test performed, according to the first aspect. Further, the physician may assign to someone else, such as a lab worker, to perform step a), and optionally step b), while performing step c), and optionally b), himself.

The inventive concept of the present disclosure may also form the basis for applying various treatment regimes.

For example, as shown in the attached figures 2-8 and 10-19, the prognoses for subjects showing low HMGCR protein levels are generally worse than those for subjects showing high HMGCR protein levels. Provided the teachings of the present disclosure, a physician may thus consider the prognosis of an HMGCR protein low subject as being so poor that a certain adjuvant treatment regimen is appropriate. The present disclosure may thus provide for accurate treatment of a previously undertreated group.

As a second aspect of the present disclosure, there is thus provided a method of treatment of a subject having an ovarian cancer, comprising:
a) evaluating the amount of HMGCR protein present in at least part of a sample from the subject and determining a sample value corresponding to said evaluated amount;
b) comparing the sample value obtained in step a) with a reference value; and, if said sample value is equal to or lower than said reference value,
c) treating said subject with an ovarian cancer treatment regimen.
   According to one embodiment, the method may comprise the additional step:
d) and if said sample value is higher than said reference value, refraining from treating said subject with the ovarian cancer treatment regimen.

In one embodiment of the method of the second aspect, the reference value of step b) may be associated with a reference prognosis and said treatment regimen of step c) may be adapted to a prognosis which is worse than or equal to the reference prognosis. In such an embodiment of the first configuration of the second aspect, the method may comprise the additional step: d) and if said sample value is higher than said reference value, treating said subject with a treatment regimen adapted to a prognosis which is better than the reference prognosis, for which the appropriate treatment regimen may be no treatment.

The physician responsible for the treatment according to the second aspect may assign to someone else, such as a lab worker, to perform step a), and optionally step b), while performing step c), and optionally b), himself.

The method of treatment may be limited to the decision-making and treatment. Thus, as a configuration of the second aspect, there is provided a method of treatment of a subject having an ovarian cancer, comprising:
α) comparing a sample value corresponding to a level of HMGCR protein in a sample from the subject with a reference value; and, if said sample value is equal to or lower than said reference value,
β) treating said subject with an adjuvant ovarian cancer treatment regimen.

Numerous ways of obtaining a sample value corresponding to a level of HMGCR protein in a sample from a subject are described in the present disclosure.

As HMGCR is believed to be the rate-limiting enzyme of the mevalonate pathway, the findings presented in the present disclosure add further evidence to the importance of this pathway in tumor development and progression. While HMGCR inhibitors, also known as statins, have shown great efficacy in the treatment of hypercholesterolemia and cardiovascular disease, their role in oncology remains relatively unproven. Despite an ever-growing body of literature describing the anti-neoplastic properties of statins, epidemiologic data regarding their preventive effect against cancer in general and ovarian cancer in particular remain inconclusive. Thus, there remains a need within the art for a tool for identifying the ovarian cancer subjects that benefit from treatment with statins.

HMGCR activity in tumor cells has been shown to be elevated and dysregulated. For example, HMGCR activity in leukemia cells (Vitols S et al (1994) Blood, 84:2689-98, Yachnin S et al (1984) Blood, 63:690-3) and lung carcinoma cells (Bennis F et al (1993) Int J Cancer, 55:640-5) were found to be 3-8-fold and 2-fold higher, respectively, than in normal cells. Furthermore, statin induced mevalonate depletion has been shown to result in an adaptive induction of HMGCR expression in chinese hamster ovary cells (Goldstein JL et al (1990) Nature, 343:425-30) and MCF-7 breast cancer cells (Duncan RE et al (2005) Cancer Lett, 224:221-8). Treatment of MCF-7 cells with mevastatin resulted in a 10- to 15-fold induction of HMGCR activity in association with a 2.5- to 3.5-fold induction of HMGCR mRNA expression (Duncan RE et al (2005) Cancer Lett, 224:221-8). Accordingly, the inventors conclude that treatment with statins may increase tumor specific HMGCR expression *in vivo.*

*In vitro* data demonstrate that statins induce apoptosis and inhibit tumor formation in soft agar in ovarian cancer cells via activation of the JNK pathway and pro-apoptotic proteins such as Bim (Liu H et al (2009) Cancer Chemother Pharmacol, 63:997-1005). Additionally statin induced suppression of RhoA has been shown to inhibit peritoneal dissemination of ovarian cancer cells *in vivo* (Horiuchi A et al (2008) Cancer Sci, 99:2532-9).

Given the discussion above and the results presented herein, the inventors believe that statin treatment is particularly relevant for HMGCR low subjects.

As a third aspect of the present disclosure, there is thus provided a method for determining whether statin treatment is suitable for a mammalian subject having an ovarian cancer, comprising the steps of:
a) evaluating an amount of HMGCR protein present in at least part of a sample earlier obtained from the subject, and determining a sample value corresponding to said evaluated amount;
b) comparing the sample value obtained in step b) with a reference value; and,
   if said sample value is lower than or equal to said reference value,
c) concluding that statin treatment is suitable for the subject.

A treatment being "suitable for" a subject refers to the case wherein the benefits of the treatment compensate for the disadvantages of the same. The benefits of the treatment refers to a higher probability of survival or recovery if undergoing the treatment than if not undergoing the treatment. The disadvantages of the treatment refers to the drawbacks, such as side-effects, pain or other inconveniences and costs.

Further, as a variant of the third aspect of the present disclosure, there is provided a method of treatment of a mammalian subject having an ovarian cancer, comprising the steps of:
a) evaluating an amount of HMGCR protein present in at least part of a sample from the subject, and determining a sample value corresponding to said evaluated amount;
b) comparing the sample value obtained in step b) with a reference value; and,
   if said sample value is lower than or equal to said reference value,
c) treating said subject with a statin treatment.

Normally, the statin treatment of step c) is administration of one or more statins to the subject.

The statin of the present disclosure may be selected from lipophilic/hydrophobic statins and hydrophobic statins. The lipophilic/hydrophobic statins comprise fluvastatin, lovastatin, simvastatin, atorvastatin and cerivastatin. The hydrophobic statins comprise pravastatin and rosuvastatin.

Regarding step a) of the methods of the present disclosure, an increase in the amount of HMGCR protein typically results in an increase in the sample value, and not the other way around. However, in some embodiments, the evaluated amount may correspond to any of a predetermined number of discrete sample values. In such embodiments, a first amount and a second, increased, amount may correspond to the same sample value. In any case, an increase in the amount of HMGCR protein will not result in a decrease in the sample value in the context of the present disclosure.

However inconvenient, but in an equivalent fashion, the evaluated amounts may be inversely related to sample values if the qualification between step b) and c) is inverted. For example, the qualification between step b) and c) is inverted if the phrase "if the sample value is lower than or equal to the reference value" is replaced with "if the sample value is higher than or equal to the reference value".

In the context of the present disclosure, "prognosis" refers to the prediction of the course or outcome of a disease and its treatment. For example, prognosis may also refer to a determination of chance of survival or recovery from a disease, as well as to a prediction of the expected survival time of a subject. A prognosis may specifically involve establishing the likelihood for survival of a subject during a period of time into the future, such as three years, five years, ten years or any other period of time. A prognosis may further be represented by a single value or a range of values.

Further, in the context of the methods of the present disclosure, "earlier obtained" refers to obtained before the method is performed. Consequently, if a sample earlier obtained from a subject used in a method, the method does not involve obtaining the sample from the subject, i.e., the sample was previously obtained from the subject in a step separate from the method.

All the methods and uses of the present disclosure, except the methods of treatment, may be carried out entirely *in vitro.*

Further, in the context of the present disclosure, "a mammalian subject having an ovarian cancer" refers to a mammalian subject having a primary or secondary ovarian tumor or a mammalian subject which has had an ovarian tumor removed, wherein the removal of the tumor refers to killing or removing the tumor by any appropriate type of surgery or therapy. In the method and use aspects of the present disclosure, "a mammalian subject having an ovarian cancer" also includes the cases wherein the mammalian subject is suspected of having an ovarian cancer at the time of the performance of the use or method and the ovarian cancer diagnosis is established later.

Further, in the context of the present disclosure, the "reference value" refers to a predetermined value found to be relevant for making decisions or drawing conclusions regarding the prognosis or a suitable treatment strategy for the subject.

Also, in the context of the present disclosure, a reference value being "associated" with a reference prognosis refers to the reference value being assigned a corresponding reference prognosis, based on empirical data and/or clinically relevant assumptions. For example, the reference value may be the average HMGCR protein value in a relevant group of subjects and the reference prognosis may be an average survival in the same group. Further, the reference value does not have to be assigned to a reference prognosis directly derived from prognosis data of a group of subjects exhibiting the reference value. The reference prognosis may for example correspond to the prognosis for subjects exhibiting the reference value or lower. That is, if the reference value is 1 on a scale from 0 to 2, the reference prognosis may be the prognosis of the subjects exhibiting the values 0 or 1. Consequently, the reference prognosis may also be adapted to the nature of the available data. As further discussed above, the reference prognosis may be further adapted to other parameters as well.

Step a) of the methods of the above aspects involve evaluating the amount of HMGCR protein present in at least part of the sample, and determining a sample value corresponding to the amount. The "at least part of the sample" refers to a relevant part or relevant parts of the sample for establishing the prognosis or drawing conclusions regarding suitable treatments. The person skilled in the art understands which part or parts that are relevant under the circumstances present when performing the method. For example, if evaluating a sample comprising cells, the skilled person may only consider the tumor cells, or only the cytoplasms of tumor cells, of the sample.

Further, in step a) an amount is evaluated and a sample value corresponding to the amount is determined. Consequently, an exact measurement of the amount of HMGCR protein is not required for obtaining the sample value. For example, the amount of HMGCR protein may be evaluated by visual inspection of a stained tissue sample and the sample value may then be categorized as a e.g. high or low based on the evaluated amount.

The person skilled in the art understands how to perform such evaluation and determination. Further, the present disclosure provides guidance for such evaluation and determination.

The treatment regimen of the present disclosure may be an adjuvant and/or a neo-adjuvant therapy.

The general strategy is that a more comprehensive treatment is applied if a subject is found to be HMGCR protein low than if the subject is found to be HMGCR protein high.

The neo-adjuvant therapy may for example be chemotherapy. Neo-adjuvant chemotherapy may be particularly relevant for a subject having a widespread tumors/a heavy tumor load.

Appropriate adjuvant therapies are primarily chemotherapies. Radiation therapy may be a suitable complement to chemotherapy in some cases, such as after tumor recurrence. The adjuvant treatment may thus be chemotherapy in combination with radiation therapy.

For example, if the subject has a stage I (1), such as la (1 a), ovarian cancer, the adjuvant treatment regimen may be chemotherapy. That means that according to some of the above methods, a chemotherapeutic agent is administered if the subject is HMGCR protein low. If, however, the subject is HMGCR protein high, the treatment with the chemotherapeutic agent may be considered unnecessary, and therefore, not administered to the subject. In the latter case, surgery may be considered sufficient.

As another example, if the subject has a stage II (2), III (3) or IV (4) ovarian cancer, the treatment regimen may be a combination of two or more chemotherapeutic agents. That means that according to some of the above methods, the combination is administered if the subject is HMGCR protein low. If, however, the subject is HMGCR protein high, the combination may be considered unnecessary, and therefore, not applied to the subject. In the latter case, treatment with one therapeutic agent only may be considered sufficient. An example of such a combination is a platinum based drug, such as carboplatin, and a taxan, such as paclitaxel.

Alternatively, the combination is applied in both cases, however in a relatively high dose if the subject is HMGCR protein low and in a relatively low dose if the subject is HMGCR protein high. Here, the "relatively high dose" is high relative the "relatively low dose". For example, a dose considered to be normal within the art may be decreased if a sample value from the subject is higher than the reference value or increased if the sample value from the subject is lower than or equal to the reference value.

Consequently, some of the methods of the present disclosure may yield information which forms the basis of a personalized treatment regimen.

The adjuvant or neo-adjuvant treatment regimen of the present disclosure may comprise treatment with one or more of the platinum-based drugs, such as carboplatin, paraplatin and cisplatin. Today, most first line ovarian cancer adjuvant treatment regimens comprise treatment with a platinum-based drug. Further, the adjuvant or neo-adjuvant treatment regimen of the present disclosure may comprise treatment with one or more drugs selected from taxanes (such as paclitaxel and docetaxel), gemcitabine, topoisomerase inhibitors (such as topotecan) and anthracyclins (such as doxorubicin). One or more of the latter drugs may be combined with one or more of the platinum based drugs, which is further discussed above.

The adjuvant chemotherapy of the present disclosure may for example be administered intraperitoneally.

The adjuvant or neo-adjuvant treatment regimen of the present disclosure may also comprise immunotherapy or radio-immunotherapy. Such therapies may for example be combined with one or more chemotherapies mentioned above. Examples of monoclonal antibodies that may be used in the immunotherapy or radio-immunotherapy are listed in tables I-V of the review article by Oei and co-workers (Oei, A.M. et al (2008) Int J Onc 32:1145-1157).

The data presented under Examples below are based on subjects having epithelial ovarian cancer. In embodiments of the present disclosure, the ovarian cancer may thus be epithelial ovarian cancer.

Further, the subjects behind the data in Examples, Sections 2 and 4 had all received adjuvant treatment with a platinum based drug, i.e. cisplatin or carboplatin. In embodiments of the present disclosure, the subject may thus be receiving platinum-based drugs or be scheduled for treatment with platinum-based drugs.

Although the level of HMGCR protein expression appears to be prognostically relevant independent of differentiation grade (see figures 2-5, 8, 10-13, 15-17 and 19), the prognostic indication is shown herein to be particularly accentuated in subjects having moderately differentiated (grade 2/intermediate) ovarian cancers (see figures 6A, 7A, 14 and 18) or poorly or moderately differentiated (grade 2 or 3) ovarian cancers (see figures 6B and 7B). Thus, in embodiments of the present disclosure, the ovarian cancer may be selected from poorly and moderately differentiated ovarian cancers. Here, the three-step differentiation grade scale of well (1)/moderately (2)/poorly (3) differentiated is used, which is well known to the skilled person.

Further, although the level of HMGCR protein expression appears to be prognostically relevant independent of stage (see figures 2-7, 10, 12, 14 and 17-19), it is shown herein that the prognostic indication may be particularly accentuated in subjects having cancers in certain stages, such as stage 3 (fig 8), stage 2 or 3 (figures 11, 13 and 15) or stage < 4 (figure 16).

Unless otherwise indicated, "stage" refers to stage according to the Federation of Gynecology and Obstetrics (FIGO) classification in the present disclosure.

Thus, in embodiments of the present disclosure, the stage of the ovarian cancer may be selected from 1, 2 and 3. In some embodiments, the stage may be 2 or 3 and in other 2. Further, in some embodiments, it may be 1 or 2.

Also, although the level of HMGCR protein expression appears to be prognostically relevant independent of ovarian cancer type (see figures 2-6, 8 and 10-16), the prognostic indication is shown herein to particularly accentuated in subjects serous (see figures 7, 17 and 18) or endometroid (see fig 19) ovarian cancer. Thus, in embodiments of the present disclosure, the ovarian cancer is selected from serous and endometroid ovarian cancers.

It follows that in embodiments of the first aspect, the first and the second group may consist of subjects having cancers of the same stage, grade and/or type as the subject of the method.

In embodiments of the first and second aspect, the prognosis may be a probability of survival. As explained in the background section, there are several ways to measure "survival". The survival of the first and second aspects may for example be overall survival, recurrence free survival or ovarian cancer specific survival. Further, the "survival" may be measured over different periods, such as five, ten or 15 years. Accordingly, the survivals may be five-year, ten-year or 15-year survivals. Where a reference prognosis is employed, it is of the same type as the prognosis of the subject.

In embodiments of the methods of the above aspects, the sample may be a body fluid sample. For example, the body fluid sample may be selected from the group consisting of blood, plasma, serum, cerebral fluid, urine, lymph and exudate. Alternatively, the sample may be a cytology sample or a stool sample.

The level of HMGCR protein expression may preferably be measured intracellularly. Thus, the body fluid, cytology or stool sample may for example comprise cells, such as tumor cells.

In further embodiments of the methods of the above aspects, the sample may be a tissue sample, such as an ovarian tissue sample, such as an ovarian tumor tissue sample, e.g, from a surgical removal of a ovarian tumor.

Further, the inventors have noted that cytoplasmic expression of HMGCR protein is particularly relevant for determining prognoses or selecting treatments. Thus, the evaluation of step a) may be limited to the cytoplasms of cells, such as tumor cell, of said sample. Consequently, when a tissue sample is examined, only the cytoplasms of tumor cells may be taken into consideration. Such examination may for example be aided by immunohistochemical staining.

A sample value of HMGCR protein being higher than the reference value, or a subject from which such sample value is obtained, is sometimes referred to herein as being "HMGCR protein high". Further, a sample value of HMGCR protein being lower than, or equal to, the reference value, or a subject from which such sample value is obtained, is sometimes referred to herein as being "HMGCR protein low".

In the context of the present disclosure, the terms "sample value" and "reference value" are to be interpreted broadly. The quantification of HMGCR protein to obtain these values may be done via automatic means, via a scoring system based on visual or microscopic inspection of samples, or via combinations thereof. However, it is also possible for a skilled person, such as a person skilled in the art of histopathology, to determine the sample and reference values merely by inspection, e.g., of tissue slides that have been stained for HMGCR protein expression. The determination of the sample value being higher than the reference value may thus correspond to the determination, upon visual or microscopic inspection, that a sample tissue slide is more densely stained and/or exhibit a larger fraction of stained cells than is the case for a reference tissue slide. The sample value may also be compared to a reference value given by a literal reference, such as a reference value described in wording or by a reference picture. Consequently, the sample and/or reference values may in some cases be mental values that the skilled person determines upon inspection and comparison.

One or more of the steps of the methods of the present disclosure may be implemented in an apparatus. For example, step a) and optionally step b) may be performed in an automatic analysis apparatus, and such apparatus may be based on a platform adapted for immunohistochemically analysis. As an example, one or more tumor tissue sample(s) from the subject in question may be prepared for imunohistochemical analysis manually and then loaded into the automatic analysis apparatus, which gives the sample value of step a) and optionally also performs the comparison with the reference value of step b). The operator performing the analysis, the physician ordering the analysis or the apparatus itself may then draw the conclusion of step c). Consequently, software adapted for drawing the conclusion of step c) may be implemented on the apparatus.

A reference value, found to be relevant for establishing prognosis or making treatment decisions regarding ovarian cancer subjects, for use as comparison with the sample value from the subject, may be provided in various ways. With the knowledge of the teachings of the present disclosure, the skilled artisan can, without undue burden, provide relevant reference values for performing the methods of the present disclosure.

The person performing the methods of the above aspects may, for example, adapt the reference value to desired information. For example, the reference value may be adapted to yield the most significant prognostic information, e.g., the largest separation between the HMGCR protein high survival curve and the HMGCR protein low survival curve (see the figures), which corresponds to the largest difference in survival between the first and the second group of the first aspect. Alternatively, the reference value may be selected such that a group having particularly good prognoses or a particularly poor prognosis is singled out.

In embodiments of the methods of the above aspects, the reference value may correspond to the amount of HMGCR protein expression in a healthy tissue, such as healthy ovarian tissue, or stroma tissue of the subject of the method. As another example, the reference value may be provided by the amount of HMGCR protein expression measured in a standard sample of normal tissue from another, comparable subject. As another example, the reference value may be provided by the amount of HMGCR protein expression measured in a reference sample comprising tumor cells, such as a reference sample of tumor tissue, e.g., ovarian cancer tissue. The amount of protein expression of the reference sample may preferably be previously established. Consequently, the reference value may be provided by the amount of HMGCR protein measured in a reference sample comprising cells expressing a predetermined amount of HMGCR protein.

Further, the reference value may for example be provided by the amount of HMGCR protein expression measured in a reference sample comprising cell lines, such as cancer cell lines, expressing a predetermined, or controlled, amount of HMGCR protein. The person skilled in the art understands how to provide such cell lines, for example guided by the disclosure of Rhodes et al. (2006) The biomedical scientist, p 515-520.

Consequently, in embodiments of the methods of the present disclosure, the reference value may be a predetermined value corresponding to the amount of HMGCR protein expression in a reference sample.

However, as discussed further below, the amount of HMGCR protein in the reference sample does not have to directly correspond to the reference value. The reference sample may also provide an amount of HMGCR protein that helps a person performing the method to assess various reference values. For example, the reference sample(s) may help in creating a mental image of the reference value by providing a "positive" reference value and/or a "negative" reference value.

One alternative for the quantification of HMGCR protein expression in a sample, such as the sample earlier obtained from the subject or the reference sample, is the determination of the overall staining intensity of the sample. The intensity may for example be: a "cellular intensity", wherein the HMGCR protein expression of the whole cells is taken into account; a "cytoplasmic intensity", wherein the HMGCR protein expression of only the cytoplasms of the cells is taken into account, or a "nuclear intensity", wherein the HMGCR protein expression of only the nuclei of the cells is taken into account. Cytoplasmic and nuclear intensity is subjectively evaluated in accordance with standards used in clinical histopathological diagnostics. Outcome of a cytoplasmic intensity determination may be classified as: absent = no overall immunoreactivity in the cytoplasms of relevant cells of the sample, weak = faint overall immunoreactivity in the cytoplasms of relevant cells of the sample, moderate = medium overall immunoreactivity in the cytoplasms of relevant cells of the sample, or strong = distinct and strong overall immunoreactivity in the cytoplasms of relevant cells of the sample. In some embodiments, the weak and moderate values may be combined into a weak/moderate value (see also Examples, section 2 and 3). The person skilled in the art understands which cells that are relevant under the conditions present when performing the method and may determine a nuclear or cytoplasmic intensity based on his general knowledge and the teachings of the present disclosure. The relevant cells may for example be tumor cells. Further, the skilled artisan understands how to perform corresponding measurements employing the "cellular intensity".

Another alternative for the quantification of HMGCR protein expression in a sample, such as the sample earlier obtained from the subject or the reference sample, is the determination of the fraction of cells in the sample that exhibit HMGCR protein expression over a certain level. The fraction may for example be: a "cellular fraction", wherein the HMGCR protein expression of the whole cells is taken into account; a "cytoplasmic fraction", wherein the HMGCR protein expression of only the cytoplasms of the cells is taken into account; or a "nuclear fraction", wherein the HMGCR protein expression of only the nuclei of the cells is taken into account. The cytoplasmic fraction may for example be classified as < 2 %, 2 - 25 %, > 25 - 75 % or > 75 % immunoreactive cells of the relevant cell population. The "cytoplasmic fraction" corresponds to the percentage of relevant cells in a sample that exhibits a positive staining in the cytoplasm, wherein a medium or distinct and strong immunoreactivity in the cytoplasm is considered positive and no or faint immunoreactivity in the cytoplasm is considered negative. The person skilled in the art of pathology understands which cells that are relevant under the conditions present when performing the method and may determine a cytoplasmic or nuclear fraction based on his general knowledge and the teachings of the present disclosure. The relevant cells may for example be tumor cells. Further, the skilled artisan understands how to perform corresponding measurements employing the "cellular fraction".

In embodiments of the methods of the above aspects, the reference value may thus be a cytoplasmic fraction, a cytoplasmic intensity or a combination thereof. Accordingly, the sample value may be a cytoplasmic fraction, a cytoplasmic intensity or a combination thereof.

As indicated figures 2-8 and 10-19, different cytoplasmic intensities may function as a relevant reference value for determining whether the prognosis for survival is relatively good or relatively poor.

Thus, in embodiments of the methods of the above aspects, the criterion for the conclusion in step c) may be a sample value, which is higher than absent cytoplasmic intensity, such as higher than weak cytoplasmic intensity, such as higher than weak/moderate cytoplasmic intensity, such as higher than moderate cytoplasmic intensity. In alternative or complementing embodiments of the methods of the above aspects, the reference value of step b) may be a moderate cytoplasmic intensity of HMGCR protein expression or lower, such as a weak/moderate cytoplasmic intensity of HMGCR protein expression or lower, such as a weak cytoplasmic intensity of HMGCR protein expression or lower, such as an absent cytoplasmic intensity.

Further, in embodiments of the methods of the above aspects, the criterion for the conclusion in step c) may be that the sample value is higher than a cytoplasmic fraction of 0 %, 1 %, 2 %, 5 %, 10 %, 15 %, 20 %, 25 %, 30 %, 35 %, 40 %, 50 %, 60 %, 70 %, 75 %, 80 %, 90 % or 95 %.

In alternative or complementing embodiments of the methods of the above aspects, the reference value of step b) may be a cytoplasmic fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower, such as 0 %.

Also, in embodiments of the methods of the above aspects, the reference value may be a function of a fraction value and an intensity value. For example, such a function may be a staining score. The "staining score" is calculated as described in Examples, Section 1 and table 1 below. For example, the reference value may be a staining score of 2 or lower, such as 1 or lower, such as 0.

The person skilled in the art realizes that another reference value which is an intensity value or a fraction value also fall within the scope of the present invention. Likewise, the person skilled in the art realizes that other combinations of fractions and intensities also fall within the scope of the present invention. Consequently, the reference value may involve two, and possibly even more, criteria.

In general, the selection of an intensity value and/or a fraction value as the reference value may depend on the staining procedure, e.g., on the employed anti-HMGCR antibody and on the staining reagents.

Further, the reference value may be an autoscore. An autoscore may be obtained as described in Examples, section 4, below. As seen in figures 4-5, an autoscore reference value of 35 results in two patient groups of different prognoses. Accordingly, the reference value of the present disclosure may be an autoscore of 15-55, such as 25-45.

Guided by the present disclosure, a person skilled in the art, e.g., a pathologist understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of HMGCR protein for establishing the appearance of a certain fraction or intensity.

However, a reference sample may not only be used for the provision of the actual reference value, but also for the provision of an example of a sample with an amount of HMGCR protein that is higher than the amount corresponding to the reference value. As an example, in histochemical staining, such as in immunohistochemical staining, the skilled artisan may use a reference sample for establishing the appearance of a stained sample having a high amount of HMGCR protein, e.g., a positive reference. Subsequently, the skilled artisan may assess the appearances of samples having lower amounts of HMGCR protein, such as the appearance of a sample with an amount of HMGCR protein corresponding to the reference value. In other words, the skilled artisan may use a reference sample to create a mental image of a reference value corresponding to an amount of HMGCR protein which is lower than that of the reference sample. Alternatively, or as a complement, in such assessments, the skilled artisan may use another reference sample having a low amount of HMGCR protein, or lacking detectable HMGCR protein, for establishing the appearance of such a sample, e.g., as a "negative reference".

For example, if a weak/moderate cytoplasmic intensity is used as the reference value, two reference samples may be employed: a first reference sample having an amount of HMGCR protein corresponding to a strong cytoplasmic intensity, which is higher than the reference value; and a second reference sample having no detectable HMGCR protein, and thus corresponding to an absent cytoplasmic intensity, which is lower than the reference value.

Consequently, in the evaluation, the skilled artisan may use a reference sample for establishing the appearance of a sample with a high amount of HMGCR protein. Such reference sample may be a sample comprising tissue expressing a high amount of HMGCR protein, such as a sample comprising ovarian tumor tissue having a pre-established high expression of HMGCR protein.

Accordingly, the reference sample may provide an example of a strong cytoplasmic intensity (CI). With the knowledge of the appearance of a sample with strong CI, the skilled artisan may then divide samples into the CI categories absent, weak, moderate and strong. This division may be further assisted by a reference sample lacking detectable HMGCR protein (negative reference), i.e., a reference sample providing an absent cytoplasmic intensity. Also, the reference sample may provide an example of a sample with a cytoplasmic fraction (CF) which is higher than 75 %. With the knowledge of the appearance of a sample with more than 75 % positive cells, the skilled artisan may then evaluate the CF of other samples having e.g., a lower percentage of positive cells. This division may be further assisted by a reference sample essentially lacking HMGCR protein (negative reference), i.e., a reference sample providing a low CF (e.g., < 5%, such as < 2%), or a NFofO.

As mentioned above, cell lines expressing a controlled amount of HMGCR protein may be used as the reference, in particular as a positive reference.

One or more pictures may also be provided as the "reference sample". For example, such a picture may show an example of a tumor tissue slide stained with a certain antibody during certain conditions and exhibiting a certain cytoplasmic intensity and/or fraction. The above discussion about the "reference sample" applies mutatis mutandis to pictures.

Further, the skilled person should recognize that the usefulness of the methods according to the above aspects is not limited to the quantification of any particular variant of the HMGCR protein present in the subject in question, as long as the protein is encoded by the relevant gene (EnsEMBL Gene ID ENSG00000113161) and presents the relevant pattern of expression. As a non-limiting example, the HMGCR protein may comprise, or consists of, a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the HMGCR protein may comprise, or consists of, a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acids may at the most be 85 % identical to a target sequence of 100 amino acids.

In some embodiments, the methods of the above aspects may comprise a further step preceding step a), which further step comprises:
obtaining biological material from the subject, excising or selecting a relevant part of the biological material to obtain said sample and optionally arranging the sample on a solid phase to facilitate the evaluation of step a). For example, such further step may comprise obtaining tissue material from the ovaries of said subject, optionally fixating the tissue material in paraffin or formalin, histo-processing the tissue material to obtain a section which constitute said sample and mounting said sample on a transparent slide, such as a glass slide, for microscopy.

In embodiments of the methods of the aspects above, the HMGCR protein may be detected and/or quantified through the application to the sample of a detectable and/or quantifiable affinity ligand, which is capable of selective interaction with the HMGCR protein. The application of the affinity ligand is performed under conditions that enable binding of the affinity ligand to any HMGCR protein in the sample.

To concretize, in embodiments of the methods of the aspects above, step a) may comprise:
a1) applying to said sample a quantifiable affinity ligand capable of selective interaction with the HMGCR protein to be evaluated, said application being performed under conditions that enable binding of said affinity ligand to HMGCR protein present in said sample;
a2) removing non-bound affinity ligand; and
a3) quantifying the affinity ligand remaining in association with said sample to evaluate said amount.

"Affinity ligand remaining in association with the sample" refers to affinity ligand which was not removed in step a2), e.g., the affinity ligand bound to the sample. Here, the binding may for example be the interaction between antibody and antigen.

However, in some embodiments, the removal of non-bound affinity ligand according to a2), e.g. the washing, is not always necessary. Thus, in some embodiments of the methods of the aspects above, step a) may comprise:
al) applying to said sample a quantifiable affinity ligand capable of selective interaction with the HMGCR protein to be evaluated, said application being performed under conditions that enable binding of said affinity ligand to HMGCR protein present in said sample;
all) quantifying the affinity bound to said sample to evaluate said amount.

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below for the sake of illustration.

Thus, in aspects and embodiments of the present disclosure, the affinity ligand may be selected from the group consisting of antibodies, fragments thereof and derivatives thereof, i.e., affinity ligands based on an immunoglobulin scaffold. The antibodies and the fragments or derivatives thereof may be isolated and/or mono-specific. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. Monoclonal antibodies of defined specificity can be produced using the hybridoma technology developed by K6hler and Milstein (Köhler G and Milstein C (1976) Eur. J. Immunol. 6:511-519). The antibody fragments and derivatives of the present disclosure are capable of selective interaction with the same antigen (e.g. HMGCR protein) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

SEQ ID NO:1 was designed for immunizations, e.g., designed to lack transmembrane regions to ensure efficient expression in *E*. *coli*, and to lack any signal peptide, since those are cleaved off in the mature protein. Further benefits of SEQ ID NO:1 are discussed below. Consequently, an antibody or fragment or derivative thereof according to the present disclosure may for example be one that is obtainable by a process comprising a step of immunizing an animal, such as a rabbit, with a protein/peptide whose amino acid sequence comprises, preferably consists of, the sequence SEQ ID NO:1. For example, the immunization process may comprise primary immunization with the protein in Freund's complete adjuvant. Also, the immunization process may further comprise boosting at least two times, in intervals of 2-6 weeks, with the protein in Freund's incomplete adjuvant. Processes for the production of antibodies or fragments or derivatives thereof against a given target are known in the art.

In the context of the present disclosure, a "mono-specific antibody" is one of a population of polyclonal antibodies which has been affinity purified on its own antigen, thereby separating such mono-specific antibodies from other antiserum proteins and non-specific antibodies. This affinity purification results in antibodies that bind selectively to its antigen. In the case of the present disclosure, the polyclonal antisera are purified by a two-step immunoaffinity based protocol to obtain mono-specific antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

Polyclonal and monoclonal antibodies, as well as their fragments and derivatives, represent the traditional choice of affinity ligands in applications requiring selective biomolecular recognition, such as in the detection and/or quantification of HMGCR protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PA and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against HMGCR protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); γ crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101 (25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

The HMGCR protein fragment SEQ ID NO:1 was designed to consist of a unique sequence with low homology with other human proteins and to minimize cross reactivity of generated affinity reagents. Consequently, in embodiments of the present disclosure, the affinity ligand may be capable of selective interaction with a polypeptide comprising or consisting of the amino acid sequence SEQ ID NO:1.

In Examples below, an antibody binding to an HMGCR epitope having the sequence SEQ ID NO:3 is employed. SEQ ID NO:3 refers to the amino acid sequence CKDNPGENARQLAR (i.e. amino acids 827-840 of EnsEMBL entry no. ENSP00000287936). This antibody resulted in strong staining. Consequently, in further embodiments of the present disclosure, the quantifiable affinity ligand may be capable of selective interaction with an HMGCR protein comprising, or consisting of, the sequence SEQ ID NO:3.

The detection and/or quantification of the affinity ligand capable of selective interaction with the HMGCR protein may be accomplished in any way known to the skilled person for detection and/or quantification of binding reagents in assays based on biological interactions. Accordingly, any affinity ligand described above may be used to quantitatively and/or qualitatively detect the presence of the HMGCR protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with HMGCR protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its HMGCR protein target may involve visualizing techniques, such as light microscopy or immunofluoresence microscopy. Other methods may involve the detection via flow cytometry or luminometry.

A biological sample, such as a tumor tissue sample (e.g., a biopsy), for example from ovarian tissue, which has been removed from the subject may be used for detection and/or quantification of HMGCR protein. The biological sample may be an earlier obtained sample. If using an earlier obtained sample in a method, no steps of the method are practiced on the human or animal body. The affinity ligand may be applied to the biological sample for detection and/or quantification of the HMGCR protein. This procedure enables not only detection of HMGCR protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from chemical reaction. Those of skill in the art are aware that a variety of different protocols can be modified in order for proper detection and/or quantification.

In embodiments of the methods of the above aspects, a biological sample may be immobilized onto a solid phase support or carrier, such as nitrocellulose or any other solid support matrix capable of immobilizing HMGCR protein present in the biological sample applied to it. Some well-known solid state support materials useful in the present invention include glass, carbohydrate (e.g., Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand specific to HMGCR protein may be applied, e.g., as described in Examples, Section 1 or 2, of the present disclosure. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art should be able to determine operative and optimal assay conditions for each determination by routine experimentation.

Consequently, in embodiments of the methods of the above aspects, the quantifiable affinity ligand of a1) or al) may be detected using a secondary affinity ligand capable of recognizing the quantifiable affinity ligand. The quantification of a3) or all) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the HMGCR protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the HMGCR protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize HMGCR protein by detection of radioactivity *in vivo* or *in vitro.* Radionuclear scanning with e.g., gamma camera, magnetic resonance spectroscopy or emission tomography function for detection *in vivo* and *in vitro,* while gamma/beta counters, scintillation counters and radiographies are also used *in vitro.*

As a fourth aspect of the present disclosure, there is provided a kit for carrying out a method according to the above aspects, which comprises:
a) a quantifiable affinity ligand capable of selective interaction with an HMGCR protein; and
b) reagents necessary for quantifying the amount of said quantifiable affinity ligand.

Various components of the kit according to the fourth aspect may be selected and specified as described above in connection with the method aspects of the present disclosure.

Thus, the kit according to the present disclosure comprises an affinity ligand against an HMGCR protein, as well as other means that help to quantify the specific and/or selective affinity ligand after it has bound specifically and/or selectively to the HMGCR protein. For example, the kit may contain a secondary affinity ligand for detecting and/or quantifying a complex formed by the HMGCR protein and the affinity ligand capable of selective interaction with the HMGCR protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g., endogenous enzyme block solution to decrease the background staining and/or counterstaining solution to increase staining contrast, that are commonly used in immunoassay reagent kits.

In embodiments of the kit aspect, the affinity ligand may be selected as described above in connection with the method aspects.

Further, in accordance with what is described above in connection with the method aspects, the detectable affinity ligand may in embodiments of the kit aspect comprise a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit according to the kit aspect may also advantageously comprise a reference sample for provision of, or yielding, the reference value to be used for comparison with the sample value. For example, the reference sample may comprise a predetermined amount of HMGCR protein. Such a reference sample may for example be constituted by a tissue sample containing the predetermined amount of HMGCR protein. The tissue reference sample may then be used by the person of skill in the art in the determination of the HMGCR expression status in the sample being studied, by manual, such as ocular, or automated comparison of expression levels in the reference tissue sample and the sample from the subject. As another example, the reference sample may comprise cell lines, such as cancer cell lines, expressing a predetermined, or controlled, amount of HMGCR protein. The person skilled in the art understands how to provide such cell lines, for example guided by the disclosure of Rhodes et al. (2006) The biomedical scientist, p 515-520. As an example, the cell lines may be formalin fixed. Also, such formalin fixed cell lines may be paraffin embedded.

The wording "reference sample for provision of the reference value" is to be interpreted broadly in the context of the present disclosure. The reference sample may comprise an amount of HMGCR protein actually corresponding to the reference value, but it may also comprise an amount of HMGCR protein corresponding to a value being higher than the reference value. In the latter case, the "high" value may be used by a person using the kit as an upper reference (positive reference) for assessing, e.g., the appearance of, a reference value which is lower than the "high" value. The person skilled in the art of immunohistochemistry understands how to do such an assessment. Further, as an alternative or a complementing example, the skilled person may use another reference sample comprising a low amount of HMGCR protein for provision of a "low" value in such an assessment, e.g., as a negative reference. This is further discussed above in connection with the method aspects.

Consequently, in embodiments of the kit aspect, the reference sample may comprise an amount of HMGCR protein corresponding to the reference value. As an example, the reference sample may comprise an amount of HMGCR protein corresponding to a moderate cytoplasmic intensity expression or lower, such as a weak/moderate cytoplasmic intensity or lower, such as a weak cytoplasmic intensity or lower, such as an absent cytoplasmic intensity.

Alternatively, or as a complement, the reference sample may comprise an amount of HMGCR protein corresponding to a cytoplasmic fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower, such as 0 %.

Further, the reference sample may comprise an amount of HMGCR protein corresponding to a staining score of 0, 1 or 2 (see table 1).

Also, the reference value may comprise an amount of HMGCR protein corresponding to an autoscore of 20-50, such as 30-40.

The provision of fraction values and intensity values is discussed above in connection with the method aspects.

Further, in alternative or complementing embodiments of the kit aspect, the kit may comprise a reference sample comprising an amount of HMGCR protein corresponding to a value being higher than the reference value. In these embodiments, the reference sample may for example comprise an amount of HMGCR protein corresponding to a cytoplasmic fraction of 75 % or higher and/or a strong cytoplasmic intensity.

In other alternative or complementing embodiments of the kit aspect, the kit may comprise a reference sample comprising an amount of HMGCR protein corresponding to a value being lower than or equal to the reference value, e.g., an absent cytoplasmic intensity and/or a cytoplasmic fraction of < 2 % HMGCR protein positive cells, such as 0 % HMGCR protein positive cells.

The kit may thus comprise: a reference sample comprising an amount of HMGCR protein corresponding to a predetermined reference value; a reference sample comprising an amount of HMGCR protein corresponding to a value being higher than a predetermined reference value; and/or a reference sample comprising an amount of HMGCR protein corresponding to a value being lower than or equal to a predetermined reference value.

Consequently, embodiments of the kit may comprise: a first reference sample comprising an amount of HMGCR protein being higher than a predetermined reference value; and a second reference sample comprising an amount of HMGCR protein being lower than or equal to the predetermined reference value.

In embodiments of the kit aspect, the reference sample may be a tissue sample, such as a tissue sample adapted to ocular or microscopic evaluation. As an example, the tissue reference sample may be fixated in paraffin or buffered formalin and/or histo-processed to sections (e.g., µm-thin sections) that are mounted on microscopic glass-slides. The tissue reference sample may be further adapted to staining with affinity ligands, such as antibodies, against an HMGCR protein.

Consequently, in embodiments of the kit aspect, the reference sample may be adapted to directly, or indirectly, provide any relevant reference value, such as any one of the reference values discussed above.

Further embodiments of the reference sample of the kit aspect are discussed above in connection with the reference values and reference samples of the method aspects.

Following the findings presented above, the inventors have realized several uses for the HMGCR protein or a fragment thereof.

Thus, as a first configuration of a fifth aspect of the present disclosure, there is provided a use of HMGCR protein, such as HMGCR protein in a sample, as a prognostic marker for a mammalian subject having an ovarian cancer.

In a similar manner, there is provided a use of HMGCR protein, such as HMGCR protein in a sample as a marker of a relatively good prognosis for a mammalian subject having an ovarian cancer.

In embodiments of the first configuration of a fifth aspect, the protein is provided in a biological sample, such as an ovarian tumor tissue sample, from a subject having an ovarian cancer.

In the context of the present disclosure, "prognostic marker" refers to something material which presence indicates a prognosis. The marker may thus be a biomarker, such as a human protein.

As a second configuration of the fifth aspect, there is provided a use of a HMGCR protein, or an antigenically active fragment thereof, for the production, selection or purification of a prognostic agent for a mammalian subject having an ovarian cancer.

In the context of the present disclosure, "prognostic agent" refers to an agent having at least one property being valuable in an establishment of a prognosis, e.g., a prognosis for a mammalian subject having an ovarian cancer. For example, the prognostic agent may be capable of selective interaction with the prognostic marker.

The prognostic agent may be an affinity ligand capable of selective interaction with the HMGCR protein, or an antigenically active fragment thereof. Examples of such affinity ligands are discussed above in connection with the method aspects.

Guided by the teachings of the present disclosure, the person skilled in the art understands how to use the HMGCR protein or the antigenically active fragment thereof in the production, selection or purification of the prognostic agent. For example, the use may comprise affinity purification on a solid support onto which the protein or fragment has been immobilized. The solid support may for example be arranged in a column. Further, the use may comprise selection of affinity ligands having specificity for the protein or fragment using a solid support onto which the polypeptide has been immobilized. Such solid support may be well plates (such as 96 well plates), magnetic beads, agarose beads or sepharose beads. Further, the use may comprise analysis of affinity ligands on a soluble matrix, for example using a dextran matrix, or use in a surface plasmon resonance instrument, such as a Biacore™ instrument, wherein the analysis may for example comprise monitoring the affinity for the immobilized protein or fragment of a number of potential affinity ligands.

Also, for the production of the prognostic agent, the HMGCR protein or antigenically active fragment thereof may be used in an immunization of an animal.

Such use may be involved in a method comprising the steps:
i) immunizing an animal using the HMGCR protein or antigenically active fragment thereof as the antigen;
ii) obtaining serum comprising the prognostic agent from the immunized animal; and, optionally,
iii) isolating the prognostic agent from the serum.

Alternatively the steps following the first step may be:
ii') obtaining cells from the immunized animal, which cells comprise DNA encoding the prognostic agent,
iii') fusing the cells with myeloma cells to obtain at least one clone, and
iv') obtaining the prognostic agent expressed by the clone.

In embodiments of the fifth aspect the amino acid sequence of the HMGCR protein may comprise or consist of a sequence selected from:
i) SEQ ID NO:2; and
ii) a sequence which is at least 85 % identical to SEQ ID NO:2.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

Further, the HMGCR protein fragment SEQ ID NO:1 is associated with a number of, which is discussed above.

Thus, in further embodiments of the fifth aspect, the amino acid sequence of the HMGCR protein may comprise or consist of a sequence selected from:
i) SEQ ID NO:1; and
ii) a sequence which is at least 85 % identical to SEQ ID NO:1.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As a sixth aspect of the present disclosure, there is provided an affinity ligand capable of selective interaction with a HMGCR protein.

Different embodiments of such an affinity ligand are discussed above in connection with the method aspects.

The affinity ligand may be used for *in vivo* diagnosis, such as *in vivo* imaging.

Thus, as a first configuration of the fifth aspect, there is provided an affinity ligand capable of selective interaction with an HMGCR protein, for *in vivo* use as a prognostic agent in a mammalian subject having an ovarian cancer.

Accordingly, in an embodiment, the affinity ligand may be for use in an *in vivo* method for establishing a prognosis for a mammalian subject having an ovarian cancer or determining whether such subject should undergo a certain treatment regimen. In such embodiments the affinity ligand may for example be labeled for enabling imaging, i.e. labeled with a detectable label. Appropriate labels for labeling affinity ligands such as antibodies are well known to the skilled person. The *in vivo* method for establishing a prognosis may for example reveal HMGCR protein expression in a tumor *in vivo,* which in turn may form the basis of a treatment decision. Various *in vivo* methods, labels and detection techniques that may be used in the context of this embodiment are further discussed above.

In a similar configuration of the fifth aspect, there is provided an affinity ligand, capable of selective interaction with an HMGCR protein, for *in vivo* evaluation an amount of HMGCR protein in a subject having an ovarian cancer. For example, the level of HMGCR expression in the ovarian tumor may be evaluated.

As a seventh aspect of the present disclosure, there is provided a use of an affinity ligand according to the sixth aspect as prognostic agent for a mammalian subject having an ovarian cancer. Consequently, the affinity ligand may be used for establishing a prognosis for an ovarian cancer subject. Such use may for example be performed *in vitro,* e.g., involving the determination of the amount of HMGCR in at least part of a sample earlier obtained from the subject.

### Examples

### 1. Tissue profiling by immunohistochemistry

### a) Material and methods

In total, 576 paraffin cores containing human tissues were analyzed using an in-house mono-specific antibody capable of selective interaction with SEQ ID NO:1 which is amino acids 742-881 of the HMGCR protein (SEQ ID NO:2; EnsEMBL entry no. ENSP00000287936). All tissues used as donor blocks for tissue microarray (TMA) production were selected from the archives at the Department of Pathology, University Hospital, Uppsala, in agreement with approval from the local ethical committee. All tissue sections used for TMA analysis were examined to determine diagnosis and to select representative areas in donor blocks. Normal tissue was defined as microscopically normal (non-neoplastic) and was most often selected from specimens collected from the vicinity of surgically removed tumors. Cancer tissue was reviewed for diagnosis and classification. All tissues were formalin fixated, paraffin embedded, and sectioned for diagnostic purposes.

The TMA production was performed essentially as previously described (Kononen J et al. (1998) Nature Med. 4:844-847; Kallioniemi OP et al. (2001) Hum. Mol. Genet. 10:657-662). Briefly, a hole was made in the recipient TMA block and a cylindrical core tissue sample from the donor block was acquired and deposited in the recipient TMA block. This was repeated in an automated tissue arrayer from Beecher Instrument (ATA-27, Beecher Instruments, Sun Prairie, CA, USA) until a complete TMA design was produced. TMA recipient blocks were baked at 42 °C for 2 h prior to sectioning.

The design of TMA:s was focused on obtaining samples from a large range of representative normal tissues, and on including representative cancer tissues. This has previously been described in detail in Kampf C et al. (2004) Clin. Proteomics 1:285-300. In brief, samples from 48 normal tissues and from 20 of the most common cancer types affecting humans were selected. In total, eight different designs of TMA blocks, each containing 72 cores of tissue with 1 mm diameter, were produced. Two of the TMA represented normal tissues, corresponding to 48 different normal tissues in triplicates from different individuals. The remaining 6 TMA:s represented cancer tissue from 20 different types of cancer. For 17 of the 20 cancer types, 12 individually different tumors were sampled, and for the remaining 3 cancer types, 4 individually different tumors were sampled, all in duplicates from the same tumor. The TMA blocks were sectioned with 4 µm thickness using a waterfall microtome (Leica), and placed onto SuperFrost® (Roche Applied Science) glass slides for IHC analysis.

Automated IHC was performed as previously described (Kampf C et al. (2004) Clin. Proteomics 1:285-300). In brief, the glass slides were incubated for 45 min in 60 °C, de-paraffinized in xylene (2 x 15 min) and hydrated in graded alcohols. For antigen retrieval, slides were immersed in TRS (Target Retrieval Solution, pH 6.0, DakoCytomation) and boiled for 4 min at 125 °C in a Decloaking chamber® (Biocare Medical). Slides were placed in the Autostainer® (DakoCytomation) and endogenous peroxidase was initially blocked with H₂O₂ (DakoCytomation). The slides were incubated for 30 min at room temperature with a primary antibody, followed by incubation for 30 min at room temperature with goat anti-rabbit peroxidase conjugated Envision®. Between all steps, slides were rinsed in wash buffer (DakoCytomation). Finally, diaminobenzidine (DakoCytomation) was used as chromogen and Harris hematoxylin (Sigma-Aldrich) was used for counterstaining. The slides were mounted with Pertex® (Histolab). The IHC analysis was performed twice; once with the in-house antibody and once with a commercially available anti-HMGCR antibody (catalog # 07-457, Upstate, Lake Placid, NY) as the primary antibody.

All immunohistochemically stained sections from the eight different TMA:s were scanned using a ScanScope T2 automated slide-scanning systems (Aperio Technologies). In order to represent the total content of the eight TMA:s, 576 digital images were generated. Scanning was performed at 20 times magnification. Digital images were separated and extracted as individual tagged image file format (TIFF) files for storage of original data. In order to be able to handle the images in a web-based annotation system, the individual images were compressed from TIFF format into JPEG format. All images of immunohistochemically stained tissue were manually evaluated under the microscope and annotated by a certified pathologist or by specially educated personnel, subsequently verified by a pathologist.

Annotation of each different normal and cancer tissue was performed using a simplified scheme for classification of IHC outcome. Each tissue was examined for representativity and immunoreactivity. The different tissue specific cell types included in each normal tissue type were annotated. For each cancer, tumor cells and stroma were annotated. Basic annotation parameters included an evaluation of i) subcellular localization (nuclear and/or cytoplasmic/membranous), ii) staining intensity (SI) and iii) fraction of stained cells (FSC). Staining intensity was subjectively evaluated in accordance to standards used in clinical histo-pathological diagnostics and outcome was classified as: absent = no immunoreactivity, weak = faint immunoreactivity, moderate = medium immunoreactivity or strong = distinct and strong immunoreactivity. The fraction of stained cells was estimated and classified as < 2 %, 2 - 25 %, > 25 - 75 % or > 75 % immunoreactive cells of the relevant cell population. Based on both the intensity and fraction of immunoreactive cells, a "staining score" was given for each tissue sample: 0 = negative, 1 = weak, 2 = moderate and 3 = strong. N.R. means that no representative tissues were present. In detail, the staining score was given according to the following criteria: 0 was given if SI = absent or weak and FSC ≤ 25%; 1 was given if SI = weak and FSC > 25% or if SI = moderate and FSC ≤ 25%; 2 was given if SI = moderate and FSC > 25% or if SI = strong and FSC ≤ 25% and SI = moderate; and finally 3 was given if SI = strong and FSC > 25%. See also table 1. The skilled artisan should recognize that this procedure is similar to a calculation of an Allred score, see e.g., Allred *et al.* (1998) Mod Pathol 11 (2), 155.

| **Table 1:** Staining score | | |
|---|---|---|
| **Staining score** | **Staining intensity** | **Fraction of stained cells** |
| 0 | absent | < 2% |
| 0 | absent | 2 - 25% |
| 0 | absent | > 25 - 75% |
| 0 | absent | > 75% |
| 0 | weak | < 2% |
| 0 | weak | 2 - 25% |
| 1 | weak | > 25 - 75% |
| 1 | weak | > 75% |
| 1 | moderate | < 2% |
| 1 | moderate | 2 - 25% |
| 2 | moderate | > 25 - 75% |
| 2 | moderate | > 75% |
| 2 | strong | < 2% |
| 2 | strong | 2 - 25% |
| 3 | strong | > 25 - 75% |
| 3 | strong | > 75% |

### b) Results

The results from tissue profiling with the mono-specific antibody showed a particular immunoreactivity in several normal tissues. Using IHC and TMA technology, 144 spots (1 mm in diameter) representing 48 different types of normal tissue were screened for expression of HMGCR. Immunoreactivity was observed mainly in cytoplasm of most tissues. Some cases showed additional nuclear or membranous positivity. Strongest staining was found in glandular epithelia. In a few cases no representative tissue (N.R.) were observed.

HMGCR protein expression was further evaluated using the two different antibodies in tissue samples from various cancer types. Table 3 shows the level of HMGCR expression in 12 different ovarian carcinoma tissues samples. HMGCR expression was observed in cytoplasm.

| **Table 3:** Expression pattern of HMGCR | | | | | | | | | | | | |
|---|---|---|---|---|---|---|---|---|---|---|---|---|
| Tissue sample | 1 | 2 | 3 | 4 | 5 | 6 | 7 | 8 | 9 | 10 | 11 | 12 |
| Staining score In-house | 3 | 2 | 2 | 1 | 1 | 1 | 1 | 1 | 0 | 0 | 0 | N.R. |
| Staining score (Upstate) | 3 | 3 | 3 | 2 | 2 | 2 | 1 | 1 | 1 | 1 | 1 | 1 |

### 2. Ovarian cancer TMA, cohort I (Dublin consecutive cohort)

### a) Material and methods

Archival formalin-fixed paraffin-embedded tissue from 76 patients surgically treated for primary invasive epithelial ovarian cancer between 1989 and 2002 was collected from the Department of Pathology at the National Maternity Hospital, Dublin, Ireland. Ethical permission was obtained from the Local Ethics Committee. The median age of patients was 52 (31-77) years. Stage (according to FIGO scale) was recorded for all specimens: 21 tumors were stage II, 54 stage III and 1 stage IV. Also grade was recorded: 12 tumors were well differentiated (grade 1), 29 were moderately differentiated (grade 2) and 35 were poorly differentiated (grade 3). The cohort included 50 high-grade serous samples, 18 endometrioid samples, 4 clear cell samples and 4 mucinous samples.

The standard surgical approach was a total abdominal hysterectomy, bilateral salpingo-oophorectomy and omentectomy with cytological evaluation of peritoneal fluid or washings. Residual disease was resected to less than 2 cm where possible. Stage and volume of residual disease (no residual disease, residual disease greater or less than 2 cm) was recorded in all cases. All patients received adjuvant chemotherapy consisting of cisplatin or carboplatin prior to 1992 and combined with paclitaxel from 1992 to 2002. No patient received neo-adjuvant chemotherapy. Median follow-up time was 4.3 years.

All 76 paraffin-embedded tumor specimens were used for tissue microarray (TMA) construction. Areas representative of invasive cancer were marked on haematoxylin and eosin- stained slides, and the TMA was constructed, using a manual tissue arrayer (MTA-1, Beecher Inc, WI). The array consisted of four cores per patient. Two 1.0 mm cores were extracted from each donor block and assembled in a recipient block. Recipient blocks were limited to approximately 100 cores each. In general, cores were taken from the peripheral part of the tumor in cases where the tumor had well-defined borders. In more diffusely growing tumors, areas with the highest tumor cell density were primarily targeted. Necrotic tissue was avoided.

Four µm sections were automatically pretreated using the PT-link system (DAKO, Copenhagen, Denmark) before being stained in a Techmate 500 (DAKO, Copenhagen, Denmark) with a polyclonal anti-HMGCR antibody (catalog # 07-457, Upstate, Lake Placid, NY) diluted 1:250, which antibody selectively interacts with a peptide consisting of the sequence SEQ ID NO:3. Cytoplasmic staining of HMGCR was assessed according to intensity (negative (0), weak/moderate (1), strong (2)), see below.

Signal was detected using the Dako REAL Detection system, which contained the secondary biotinylated goat anti-rabbit/mouse antibody, the strepavidin/ horseradish peroxidase complex and 3,30- diaminobenzidine, applied according to the manufacturer's protocol. Slides were counterstained with Mayers haematoxylin (Sigma-Aldrich, St. Louis, MO).

All samples of immunohistochemically stained tissue were manually evaluated under the microscope and annotated by a certified pathologist. Annotation of each sample was performed using a simplified scheme for classification of IHC outcome. Each tissue sample was examined for representativity and immunoreactivity.

For statistical analyses, the cytoplasmic intensity (CI) level was evaluated in accordance to standards used in clinical histo-pathological diagnostics and outcome was classified as: absent = no immunoreactivity, weak/moderate = faint to medium immunoreactivity, or strong = distinct and strong immunoreactivity. Thus, tissue annotation was essentially done as described in Examples, section 1 above, with the exception that only staining intensity was evaluated.

Based on the survival trends for individual strata, dichotomized variables were constructed for further statistical analyses. A high cytoplasmic intensity was defined as a weak/moderate or strong cytoplasmic intensity (CI > 0) and a low cytoplasmic intensity was defined as an absent cytoplasmic intensity CI = 0).

The above classification of samples was used for overall survival (OS) and recurrence free survival (RFS) analysis according to the Kaplan-Meier method, and the log-rank test was used to compare survival in different strata. All statistical tests were two-sided, and p-values of < 0.05 % were considered significant. All calculations were made with the statistical package SPSS 17.0 (SPSS Inc. Illinois, USA).

### b) Results

Initial analysis of the cohort revealed that OS for all patients was approximately 40% and RFS was approximately 29%, as seen in figure 1A and 1 B, respectively.

Immunohistochemical analysis of HMGCR expression could be performed on 71 tumor samples. The remaining cores either did not contain tumor cells or had been lost during histoprocessing. Cytoplasmic staining was observed in 46 of the 71 subjects (65 %), i.e. no HMGCR expression was observed in 25 of the tumor samples (CI = 0).

Survival analysis of the entire cohort revealed that expression of HMGCR in tumor tissues was correlated with OS and RFS (Figure 2 and 3). Further, the prognostic value was significant in a group of patients having grade 2 tumors (fig 6A) or grade 2 or 3 tumors (fig 6B). In the subgroup of serous tumors this finding was even more pronounced (fig 7A and 7B). Expression of HMGCR also indicated a better survival for patients having grade 3 serous tumors, as seen in figure 7C. For patients diagnosed with stage 3 EOC, expression of HMGCR indicates a better survival than the lack thereof (figure 8).

In conclusion, for a patient diagnosed with EOC, e.g. serous adenocarcinoma, the use of an anti-HMGCR protein affinity ligand may be of significant value for establishing a prognosis, e.g. the probability of survival, such as five-year survival, as can be seen from figures 1 to 8.

### 3. Ovarian cancer TMA, cohort II (MKC_MFM prospective cohort)

### a) Material and methods

Archival formalin-fixed paraffin-embedded tissues from 154 patients surgically treated for primary invasive epithelial ovarian cancer were collected from the Pathology departments at Lund and Malmö University Hospitals, Sweden. Patients were diagnosed between 1982 and 2007. Ethical permission was obtained from the Local Ethics Committee in Lund.

The median age of patients was 63 (47-83) years. Stage (according to the FIGO scale) was recorded for all specimens: 26 tumors were stage I, 18 tumors were stage II, 75 stage III and 22 stage IV. Also grade was recorded: 8 tumors were well differentiated (grade 1), 39 were moderately differentiated (grade 2) and 107 were poorly differentiated (grade 3). The cohort included 90 cases of serous carcinoma, 35 endometrioid carcinoma, 12 mucinous samples, 9 clear cell samples, 1 Brenner tumor and 7 of unknown histological type.

Median follow-up time was 2.67 years.

All paraffin-embedded tumor specimens were used for tissue microarray (TMA) construction. Areas representative of invasive cancer were marked on haematoxylin and eosin- stained slides, and the TMA was constructed, using a manual tissue arrayer (MTA-1, Beecher Inc, WI). The array consisted of four cores per patient. Two 1.0 mm cores were extracted from each donor block and assembled in a recipient block. Recipient blocks were limited to approximately 100 cores each. In general, cores were taken from the peripheral part of the tumor in cases where the tumor had well-defined borders. In more diffusely growing tumors, areas with the highest tumor cell density were primarily targeted. Necrotic tissue was avoided.

Four µm sections were automatically pretreated using the PT-link system (DAKO, Copenhagen, Denmark) before being stained in a Techmate 500 (DAKO, Copenhagen, Denmark) with a polyclonal anti-HMGCR antibody (Upstate, Lake Placid, NY) diluted1:250 , which antibody selectively interacts with a peptide consisting of the sequence SEQ ID NO:3. Cytoplasmic staining of HMGCR was assessed according to intensity (negative (0), weak/moderate (1), strong (2)), see section 4a above.

Signal was detected using the Dako REAL Detection system, which contained the secondary biotinylated goat anti-rabbit/mouse antibody, the strepavidin/ horseradish peroxidase complex and 3,30- diaminobenzidine, applied according to the manufacturer's protocol. Slides were counterstained with Mayers haematoxylin (Sigma-Aldrich, St. Louis, MO).

All samples of immunohistochemically stained tissue were manually evaluated under the microscope and annotated by a certified pathologist. Annotation of each sample was performed using a simplified scheme for classification of IHC outcome. Each tissue sample was examined for representativity and immunoreactivity.

Based on the survival trends for individual strata, dichotomized variables were constructed for further statistical analyses. A high cytoplasmic intensity was defined as strong cytoplasmic intensity (CI < 2) and a low cytoplasmic intensity was defined as an absent or weak/moderate cytoplasmic intensity CI = 0).

The above classification of samples was used for overall survival (OS) and ovarian cancer specific survival (OCSS) analysis according to the Kaplan-Meier method, and the log-rank test was used to compare survival in different strata. All statistical tests were two-sided, and p-values of < 0.05 % were considered significant. All calculations were made with the statistical package SPSS 17.0 (SPSS Inc. Illinois, USA).

### b) Results

Initial analysis of the cohort revealed that OS for all patients was approximately 32% and OCSS was approximately 34%, as seen in figure 9A and 9B, respectively.

Immunohistochemical analysis of HMGCR expression could be performed on all the154 tumor samples. Cytoplasmic staining was observed in 125 of the 154 subjects (81 %), i.e. no HMGCR expression was observed in 29 of the tumor samples (CI = 0).

Survival analysis of the entire cohort revealed that expression of HMGCR in tumor tissues was significantly correlated with OS and OCSS (Figure 10B and 12B). Further, the correlation was also significant when analyzing the group of patients having stage 2 or 3 tumors as seen in figure 11B and 13B. This subgroup resembles the material in cohort I, and may be a relevant group for comparison. The similar scenario was observed for the group of subjects having tumors in a stage which is lower than 4 (fig 15).

Regarding the group of patients having grade 2 tumors, a trend of better overall or ovarian cancer specific survival for HMGCR high patients was observed (fig 14).

The subgroup of serous tumors showed a trend towards better survival for patients with strong HMGCR expression (figure 17), and if analyzing only grade 2 tumors the trend became significant, as seen in figure 18A and 18B. For the subgroup of endometrioid tumors, strong HMGCR expressions were highly associated with survival (figure 19).

In conclusion, for a patient diagnosed with EOC, e.g. serous- or endometrioid adenocarcinoma, the use of an anti-HMGCR protein affinity ligand may be of significant value for establishing a prognosis for a patient, e.g. the probability of survival, such as five-year survival, as can be seen from figures 9-19.

### 4. Automated analysis

### a) Materials and methods

In addition to manual evaluation, the Dublin cohort (cohort I) was analyzed by an automatic analysis method., The Aperio ScanScope XT Slide Scanner (Aperio Technologies, Vista, CA) system was used to capture digital images. Images were captured at 20X the average file size for a full TMA scanned was 200 megabytes. Images were stored as multilayered tagged image format files (TIFF). Digital slides were viewed using lmagescope (Aperio) and deemed suitable for analysis. Slides were de-arrayed to visualize individual tissue cores using Spectrum (Aperio). A number of different algorithms were used to quantify nuclear, cytoplasmic and membranous staining. Initially Genie (Aperio) pattern recognition software was used to identify tumor from stroma. Tumor-specific 3,3'-Diaminobenzidine (DAB) signal was then quantified using a color deconvolution algorithm (Aperio).

A markup image of each core was also generated and evaluated to confirm the accuracy of each algorithm. The output of the algorithm is an average intensity value for DAB signal for each core (ranging 0-255) and the percentage tumor cells displaying cytoplasmic expression for each core. An autoscore (AS) was calculated by multiplying the percentage positive tumor cells by the average RGB intensity for each core. This automated analysis method was recently described by Brennan and co-workers (Brennan et al (2008) Clin Can Res 2008 14(9) 2681-9).

### b) Results

Automated analysis was used in this study to develop a quantitative scoring model for HMGCR expression in EOC. The approach adopted in this study differed from previous experiments as pattern recognition software (Genie, Aperio) was initially used to identify tumor from stroma and then tumor-specific HMGCR expression was quantified using a positive pixel count algorithm. The output of the algorithm was staining intensity and percentage positive tumor cells. An excellent correlation was seen between manual and automated analysis of staining intensity (r = 0.61, p < 0.001).

Automated analysis was used to develop a HMGCR autoscore, which combined intensity and percentage positive tumor cells. As specimens were arrayed in quadruplicate a median HMGCR autoscore was calculated for each tumor. HMGCR autoscore was then dichotomized using the 25th percentile (corresponding to an autoscore value of 35) as a threshold. Kaplan Meier analysis of the HMGCR as a dichotomized value demonstrated that increased levels of HMGCR protein expression were significantly associated with an improved RFS (p = 0.012) (Fig. 4B). A high HMGCR autoscore was associated with a trend towards an improved OS (p = 0.131) (Fig. 4A). Further, analysis of five year RFS revealed a significantly (p=0.027) better survival for HMGCR high patients with grade 1 or grade 2 tumors (p=0.017) (Fig. 5A and 5B).

Consequently, the methods of the present disclosure may be performed using automated immunohistochemical analysis.

### 5. A non-limiting example

Following the establishment of an ovarian cancer diagnosis in a patient, a tumor tissue sample from the patient is obtained. The tumor tissue sample may be obtained from a biopsy performed earlier during the diagnosis of the cancer or from a specimen from an earlier surgical removal of the tumor. Further, for the provision of a "negative reference", a sample is taken from archival material comprising tissue having low, or essentially lacking, HMGCR protein expression. Such archival tissue may for example be ovarian cancer tissue having a pre-established low HMGCR protein expression level. Further, for the provision of a "positive reference", a sample is taken from archival material comprising tissue having high HMGCR protein expression, such as ovarian cancer tissue having a pre-established high HMGCR protein expression level.

The sample material is fixated in buffered formalin and histo-processed in order to obtain thin sections (4 µm) of the of the sample material.

Immunohistochemistry is performed as described in Examples, Section 1. One or more sample sections from each sample is/are mounted on glass slides that are incubated for 45 min in 60 °C, de-paraffinized (if the sample in question was paraffinized) in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple sample sections is to increase the accuracy of the results.

A primary HMGCR protein specific antibody (e.g. the Upstate antibody employed in Examples, section 1) is added to the slides and incubated for 30 min in room temperature, followed by 30 min of incubation in room temperature with a labeled secondary antibody; e.g. goat-anti-rabbit peroxidase conjugated Envision®. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab) mounting media.

As a tool to validate the staining procedure, two control cell-lines may be used; e.g. one slide with cells expressing HMGCR protein (positive cell line) and one slide having cells with indistinct weak or no HMGCR protein expression (negative cell line). The skilled artisan understands how to provide such cell lines, for example guided by the disclosure of Rhodes et al. (2006) The biomedical scientist, p 515-520. The control-line slides may be simultaneously stained in the same procedure as the other slides, i.e. incubated with the same primary and secondary antibodies.

For example, the tumor tissue slides from the subject, the staining reference slides, and optionally, the slides with control cell-lines, may be scanned in a light microscope using a ScanScope T2 automated slide scanning system (Aperio Technologies) at x20 magnification. However, this scanning step is not necessary, but may make the procedure easier if, for example, the preparation and staining of the slides and the evaluation of the stained slides (see below) are performed at different locations or by different persons.

If control cell-lines are used, these are inspected to validate the staining procedure. If the cell-lines display staining results outside acceptable criteria, e.g. staining artifacts recognized by the skilled artisan, the staining of the tissue samples is considered invalid and the whole staining procedure is repeated with new slides. If the positive and negative cell-lines display strong staining intensity and indistinct weak or no staining intensity, respectively, the staining is considered as valid.

The stained sample slide(s) from the tumor tissue sample from the patient is/are evaluated manually by visual inspection, and the cytoplasmic intensity (CI) of the ovarian cancer slide(s) is/are graded as described in Examples, Section 2. In the grading, the cytoplasmic intensity (CI) is subjectively classified as: absent = no immunoreactivity, weak/moderate = faint or medium immunoreactivity or strong = distinct and strong immunoreactivity. The person performing the evaluation and grading is aided by visual inspection of the stained positive and negative reference slides.

The CI value(s) from the tumor tissue sample from the patient is/are then compared to a reference value. If more than one sample slide are evaluated and thereby more than one CI value are obtained, the sample value that is compared to the reference value may be a mean or median value of the obtained CI values.

The reference value may be an absent CI. In such case it is concluded that the tested patient belongs to a group of patients having a relatively good prognosis if the sample value is a weak/moderate or strong CI and a group of patients having a relatively poor prognosis if the sample value is an absent CI. The prognoses of the respective groups may be read from dichotomized data as those presented in figure 2B or 3B, wherein the upper curve represents the group of patients having the relatively good prognosis and the lower curve represents the group of patients having the relatively poor prognosis. For example, the relatively good prognosis may be an average five-year overall survival of about 48 % and the poor prognosis may be an average five-year overall survival of about 25 % (fig 2B). Alternatively, the relatively good prognosis may be an average five-year recurrence free survival of about 39 % and the poor prognosis may be an average five-year recurrence free survival of about 12 % (fig 2B).

If the cancer of the subject is serous and of grade 2 or 3, the respective prognoses may be read from dichotomized data exclusively based on subjects having such a cancer, such as in figure 7B.

Also, the reference value may be a weak/moderate CI. In such case it is concluded that the tested patient belongs to a group of patients having a relatively good prognosis if the sample value is a strong CI and a group of patients having a relatively poor prognosis if the sample value is an absent or weak/moderate CI. The prognoses of the respective groups may be read from dichotomized data as those presented in figures 10B and 12B, wherein the upper curve represents the group of patients having the relatively good prognosis and the lower curve represents the group of patients having the relatively poor prognosis. For example, the relatively good prognosis may be an average five-year overall survival of about 58 % and the poor prognosis may be an average five-year overall survival of about 24 % (fig 10B). Alternatively, the relatively good prognosis may be an average five-year ovarian cancer specific survival of about 58 % and the poor prognosis may be an average five-year ovarian cancer specific survival of about 27 % (fig 12B).

All cited material, including but not limited to publications, DNA or protein data entries, and patents, referred to in this application are herein incorporated by reference.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. Method for determining whether a mammalian subject having an ovarian cancer belongs to a first or a second group, wherein the prognosis of subjects of the first group is better than the prognosis of subjects of the second group, comprising the steps of:
a) evaluating the amount of HMGCR protein in at least part of a sample earlier obtained from the subject and determining a sample value corresponding to said evaluated amount;
b) comparing said sample value with a predetermined reference value;
and
if said sample value is higher than said reference value,
c1) concluding that the subject belongs to the first group; and
if said sample value is lower than or equal to said reference value,
c2) concluding that the subject belongs to the second group.

2. Method for determining whether a subject having an ovarian cancer is not in need of a treatment with an ovarian cancer treatment regimen, comprising the steps of:
a) evaluating the amount of HMGCR protein present in at least part of a sample earlier obtained from the subject, and determining a sample value corresponding to said evaluated amount;
b) comparing the sample value obtained in step a) with a reference value; and,
if said sample value is higher than said reference value,
c) concluding that said subject is not in need of the treatment with the ovarian cancer treatment regimen.

3. Non-treatment strategy method for a subject having an ovarian cancer, comprising:
a) evaluating the amount of HMGCR protein present in at least part of a sample earlier obtained from the subject, and determining a sample value corresponding to said evaluated amount;
b) comparing the sample value obtained in step a) with a reference value; and,
if said sample value is higher than said reference value,
c) refraining from treating said subject with an ovarian cancer treatment regimen.

4. Method according to any one of the preceding claims, wherein ovarian cancer is poorly or moderately differentiated.

5. Method according to any one of the preceding claims, wherein said ovarian cancer is in stage 1, 2 or 3 according to the Federation of Gynecology and Obstetrics (FIGO) classification.

6. Method according to any one of the preceding claims, wherein said ovarian cancer is an epithelial ovarian cancer.

7. Method according to any one of the preceding claims, wherein said ovarian cancer is selected from serous and endometroid ovarian cancer.

8. Method according to any one of the preceding claims, wherein said sample comprises tumor cells from said subject.

9. Method according to claim 8, wherein the evaluation of step a) is limited to the cytoplasms of tumor cells of said sample.

10. Method according to any one of the preceding claims, wherein step a) comprises:
al) applying to said sample a quantifiable affinity ligand capable of selective interaction with the HMGCR protein to be evaluated, said application being performed under conditions that enable binding of the affinity ligand to HMGCR protein present in the sample; and
all) quantifying the affinity ligand bound to said sample to evaluate said amount.

11. Kit for carrying out a method according to any one of the preceding claims, which comprises
a) a quantifiable affinity ligand capable of selective interaction with an HMGCR protein; and
b) reagents necessary for quantifying the amount of said quantifiable affinity ligand.

12. Kit according to any one of claim 11, further comprising at least one reference sample for provision of a reference value.

13. Use *in vitro* of an HMGCR protein as a prognostic marker for ovarian cancer.

14. Use *in vitro* of an HMGCR protein, or an antigenically active fragment thereof, for the production, selection or purification of a prognostic agent for establishing a prognosis for a mammalian subject having an ovarian cancer.

15. Use *in vitro* of an affinity ligand capable of selective interaction with a HMGCR protein as a prognostic agent for ovarian cancer.
